# EUROPEAN PATENT APPLICATION

(11) **EP 4 162 941 A1**
(43) Date of publication of application: **12.04.2023**
(21) Application number: 21797458.3
(22) Date of filing: 23.04.2021
(51) Int. Cl.: A61K 35/768, C12N 7/00, C12N 7/01, A61P 35/00, C12N 15/38, C12N 15/33, C12N 15/66

(54) **ISOLATED RECOMBINANT ONCOLYTIC POXVIRUS CAPABLE OF BEING REGULATED AND CONTROLLED BY MICRORNA AND USE THEREOF**

(30) Priority: 30.04.2020 CN 202010363270
(71) Applicant: Hangzhou Converd Co., Ltd., Hangzhou, Zhejiang 311121 (CN)
(72) Inventor: FU, Jin, Hangzhou, Zhejiang 311121 (CN); ZHANG, Rong, Hangzhou, Zhejiang 311121 (CN); WANG, Tingting, Hangzhou, Zhejiang 311121 (CN); HU, Fang, Hangzhou, Zhejiang 311121 (CN); YAN, Hui, Hangzhou, Zhejiang 311121 (CN)
(74) Representative: Ipsilon
(86) International application number: PCT/CN2021/089255
(87) International publication number: WO 2021/218802

(57) **Abstract**

Provided are an isolated recombinant oncolytic poxvirus capable of being regulated and controlled by microRNA and a use thereof. The isolated recombinant oncolytic poxvirus can be regulated and controlled by microRNA, and the expression level of the microRNA in tumor cells of a mammal is lower than that in normal cells of the same mammal. A target sequence of the microRNA is integrated in a 3'UTR region of an E10R gene in a recombinant oncolytic poxvirus genome.

## Description

### Field of the Invention

The present invention belongs to the field of biotechnology, and specifically relates to an isolated recombinant oncolytic vaccinia virus capable of being regulated by a microRNA, pharmaceutical compositions and uses thereof for drugs for the treatment of tumors and/or cancers.

### Background of the Invention

As early as the end of the 19th century, multiple viruses were found to alleviate the progression of tumor development, indicating the potential of viruses in the field of tumor therapy. Vaccinia viruses are double-stranded DNA viruses. Based on their good safety, stability, strong immune response, efficient oncolytic effect and effective spread in tumors, they have gained more and more attention in the field of tumor immunotherapy. Although vaccinia viruses are naturally tumor-targeting, wild-type viruses without genetic modification may still infect normal cells, which causes unpredicted risk. Several strains of oncolytic vaccinia viruses have been reported, including Western Reserve, Wyeth, Copenhagen, Lister, etc., to use for construction of mutants. Among these mutants, the thymus kinase (TK) gene of vaccinia virus is one of the most frequently used mutant regions. TK gene is one of the key genes involved in the viral replication process. The high expression of TK gene in tumor tissue allow the TK-deficient mutants replication in tumors, while limit replicated capacity in normal tissues. Secondly, the oncolytic effect of vaccinia viruses in host cells is closely related to the activation of the epidermal growth factor receptor (EGFR) signaling pathway. VGF secreted after the infection of cells by vaccinia viruses binds to the EGFR on the surface of infected or adjacent uninfected cells and activates EGFR/Ras signaling pathway, providing a favorable environment for vaccinia viruses to infect adjacent cells. Therefore, the VGF-deficient vaccinia virus cannot activate the EGFR/Ras pathway in normal cells, while the EGFR signaling pathway in tumor cells is activated, so that the infection of the VGF gene-deficient vaccinia virus to tumor cells is not affected, that is, tumor specificity is relatively increased. However, in clinical use, the safety of VGF-deficient and/or TK-deficient oncolytic vaccinia viruses still have been concerned when they are administered by systemically.

The China Food and Drug Administration (CFDA) approved the gene-modified oncolytic adenoviruses H101 of Shanghai Sunway Biotech Co., Ltd. for the treatment of head and neck tumors in 2005; the US FDA and EU EMA approved Amgen's gene-modified oncolytic herpes simplex virus T-Vec for the treatment of advanced malignant melanoma in 2015. However, there is currently no oncolytic vaccinia virus product in the market.

Therefore, there is still a need to develop products with improved drug efficacy, higher tumor specificity and safety in the immunotherapy of tumors and/or cancers by oncolytic viruses.

### Summary of the Invention

In order to solve the existing problems in the prior art, the present invention provides an isolated recombinant oncolytic vaccinia virus, pharmaceutical compositions and uses thereof for drugs for the treatment of tumors and/or cancers.

Specifically, the present invention provides:
(1) An isolated recombinant oncolytic vaccinia virus, wherein the recombinant oncolytic vaccinia virus is capable of being regulated by a microRNA which has a lower expression level in tumor cells of a mammal than in normal cells of the same mammal, wherein the 3'UTR region of the E10R gene in the recombinant oncolytic vaccinia virus genome is integrated with a target sequence of the microRNA.
(2) The recombinant oncolytic vaccinia virus according to (1), wherein the microRNA is selected from a group consisting of miR-9, miR-15a, miR-16, miR-26a, miR-27b, miR-29b, miR-30a, miR-32, miR-33, miR-34, miR-95, miR-101, miR-122, miR-124, miR-125a, miR-125b, miR-126, miR-127, miR-128, miR-133b, miR-139, miR-140, miR-142, miR-143, miR-145, miR-181, miR-192, miR-195, miR-198, miR-199a, miR-199b, miR-200, miR-203, miR-204, miR-205, miR-218, miR-219, miR-220, miR-224, miR-345 and miR-375; preferably miR-199a and miR-199b.
(3) The recombinant oncolytic vaccinia virus according to (1), wherein the recombinant oncolytic vaccinia virus is functionally deficient in TK gene and/or VGF gene.
(4) The recombinant oncolytic vaccinia virus according to (1), wherein the target sequence of the microRNA is repeated and comprised of 2-8 repeats.
(5) The recombinant oncolytic vaccinia virus according to (3), wherein the genome of the recombinant oncolytic vaccinia virus is integrated with an exogenous IL-21 gene, and wherein the IL-21 gene is capable of being expressed in tumor cells.
(6) The recombinant oncolytic vaccinia virus according to (3), wherein the TK gene is rendered functionally deficient via the insertion of an exogenous nucleotide sequence into the TK locus.
(7) The recombinant oncolytic vaccinia virus according to (5), wherein the exogenous IL-21 gene is inserted into the TK locus, thereby causing functional defect of the TK gene.
(8) The recombinant oncolytic vaccinia virus according to (3), wherein the VGF gene is rendered functionally deficient by being knocked out or via the insertion of an exogenous nucleotide sequence into the VGF locus.
(9) The recombinant oncolytic vaccinia virus according to (1), wherein the recombinant oncolytic vaccinia virus is the WR strain or the Wyeth strain.
(10) The recombinant oncolytic vaccinia virus according to (1), wherein the genome of the recombinant oncolytic vaccinia virus is further integrated with an exogenous screening gene which includes gpt gene and/or LacZ gene.
(11) The recombinant oncolytic vaccinia virus according to (5), wherein the exogenous IL-21 gene is derived from mouse or human.
(12) A pharmaceutical composition, wherein the pharmaceutical composition comprises the recombinant oncolytic vaccinia virus according to any one of (1)-(11) as an active ingredient, and a pharmaceutically acceptable excipient.
(13) The pharmaceutical composition according to (12), wherein the pharmaceutical composition comprises the recombinant oncolytic vaccinia virus at doses of 1 × 10⁵ to 5 × 10⁹ pfu.
(14) The pharmaceutical composition according to (12), wherein the recombinant oncolytic vaccinia virus is administered by intratumoral injection or intravenous administration.
(15) A vector for preparing the recombinant oncolytic vaccinia virus according to any one of (1) to (11).
(16) The vector according to (15), wherein the vector comprises an exogenous IL-21 gene controlled by a promoter.
(17) A host cell comprising the vector according to (15) or (16).
(18) Use of the recombinant oncolytic vaccinia virus according to any one of (1) to (11) for the preparation of drugs for the treatment of tumors and/or cancers.
(19) The use according to (18), wherein the tumors and/or cancers include lung cancer, melanoma, head and neck cancer, liver cancer, intracranial tumor, colorectal cancer, bladder cancer, breast cancer, ovarian cancer, uterus cancer, cervical cancer, gastric cancer, esophageal cancer, kidney cancer, prostate cancer, pancreatic cancer, lymphoid cancer, leukemia, bone cancer, testicular cancer and osteosarcoma.
(20) A method for treating tumors and/or cancers, comprising administering the recombinant oncolytic vaccinia virus according to any one of (1) to (11) to a tumor and /or cancer patient.
(21) The method according to (20), wherein the recombinant oncolytic vaccinia virus is administered at dose of 1 × 10⁵ to 5 × 10⁹ pfu, once per day for 1-6 consecutive days, or once every two days for 1-6 consecutive times.
(22) The method according to (20), wherein the oncolytic virus is administered by intratumoral injection or intravenous administration.
(23) The method according to (20), wherein the tumors and/or cancers include lung cancer, melanoma, head and neck cancer, liver cancer, intracranial tumor, colorectal cancer, bladder cancer, breast cancer, ovarian cancer, uterus cancer, cervical cancer, gastric cancer, esophageal cancer, kidney cancer, prostate cancer, pancreatic cancer, lymphoid cancer, leukemia, bone cancer, testicular cancer and osteosarcoma.

Compared with the prior art, the present invention has the following advantages and positive effects:
Through intensive research and experiments, the present invention selects a specific essential gene E10R from the genome of an oncolytic vaccinia virus, and proposes to insert an exogenous nucleotide sequence into the 3'UTR region (3' untranslated region) of the E10R, wherein the exogenous nucleotide sequence contains a target sequence of a specific microRNA and wherein the microRNA has a lower expression level in tumor cells than in normal cells. In this way, in normal cells infected with the oncolytic vaccinia virus, the high-expressing microRNA can target the corresponding mRNA in the 3'UTR region of the E10R in the oncolytic vaccinia virus and block the expression of E10R by degrading the mRNA or hindering its translation, thereby inhibiting the replication of the oncolytic vaccinia virus. In contrast, the oncolytic vaccinia virus infects tumor cells, the microRNA is low-expressed or not expressed, so that the expression of E10R is not inhibited, thereby maintaining the replication ability of the oncolytic vaccinia virus. Therefore, by using the characteristic that the expression level of specific microRNA in normal tissues may be different than that in tumor tissues *in vivo,* the present invention provides a novel recombinant oncolytic vaccinia virus with significant selectivity to tumor cells. The inventors have surprisingly found that the recombinant oncolytic vaccinia virus of the present invention has a significantly higher replication in various tumor cells than in normal cells, thereby having superior tumor specificity and safety, and can significantly inhibit the growth of tumor cells.

In addition, the types of microRNAs were further selected in the present invention, and it was found that a variety of microRNAs (preferably miR-199) effectively achieve the purpose of the present invention, and the recombinant oncolytic vaccinia viruses show stronger tumor selectivity and safety.

The novel recombinant oncolytic vaccinia viruses of the present invention may further include inactive TK gene and/or VGF gene, thereby achieving a further superior tumor selectivity, with significantly reduced replication in normal cells and significantly reduced killing effect on normal cells, compared with the existing oncolytic vaccinia viruses (such as TK-deficient and/or VGF-deficient vaccinia viruses).

In addition, the present invention further enables novel oncolytic vaccinia viruses carried the gene of immune modulator IL-21, so that the resulting recombinant oncolytic vaccinia viruses selectively replicate in tumor cells and express the immune modulator IL-21. In this way, the present invention fully exerts the roles of novel oncolytic vaccinia virus selectively replicating in tumor cells and killing tumor cells, causing subsequent immune response in the body, and simultaneously can also fully exert the anti-tumor immune effect of the exogenous IL-21. The present invention finds that the integration of the IL-21 gene in the oncolytic vaccinia virus is able to synergize the oncolytic killing effect of the oncolytic virus and the anti-tumor immune stimulating effect of IL-21.

Thus, the present invention provides abundant product lines with more efficiency for cancer treatment, and can provide products with superior effects, tumor-target specificity and safety.

### Brief Description of the Drawings

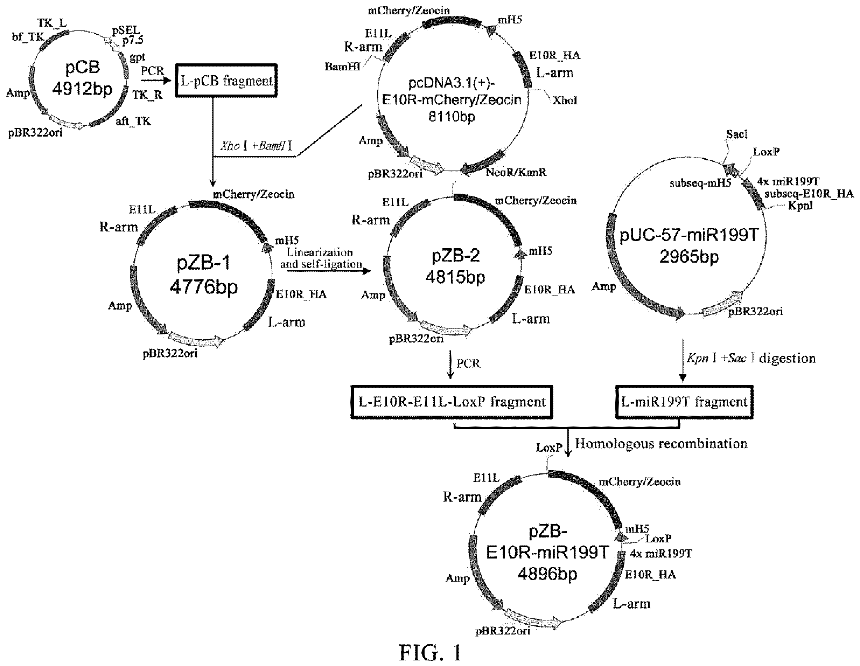

Figure 1 shows the scheme of the construction of plasmid pZB-E10R-miR199T in Preparation Example 1.

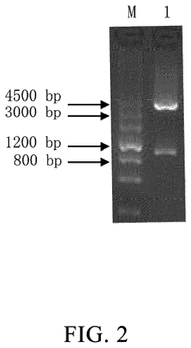

Figure 2 shows the identification results of the pZB-E10R-miR199T plasmid in Preparation Example 1; wherein the lane M is the DNA Marker III (DNA molecular weight marker III), and the lane 1 is the pZB-E10R-miR199T plasmid digested with restriction enzyme.

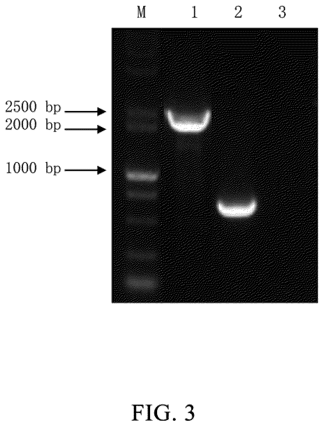

Figure 3 shows the identification results, assessed by PCR, of the monoclonal recombinant oncolytic virus of the backbone virus VSC20-mT/mCherry constructed in Preparation Example 2; wherein the lane M is the DNA molecular weight marker, the lane 1 is the VSC20-mT/mCherry, the lane 2 is the VSC20, and the lane 3 is the negative control (without DNA sample).

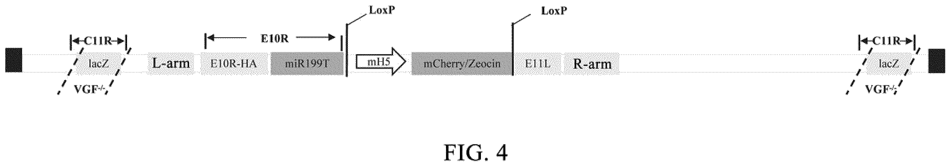

Figure 4 shows the structure of the oncolytic virus vector VSC20-mT/mCherry constructed in Preparation Example 2.

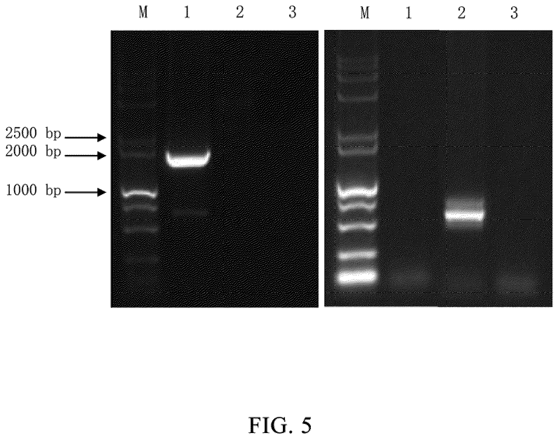

Figure 5 shows the identification results, assessed by PCR, of the monoclonal recombinant oncolytic virus of the backbone virus MiTDvv-mCherry constructed in Preparation Example 3; wherein the lane M is the DNA molecular weight marker, the lane 1 is the MiTDvv-mCherry, the lane 2 is the VSC20-mT/mCherry, and the lane 3 is the negative control (without DNA sample).

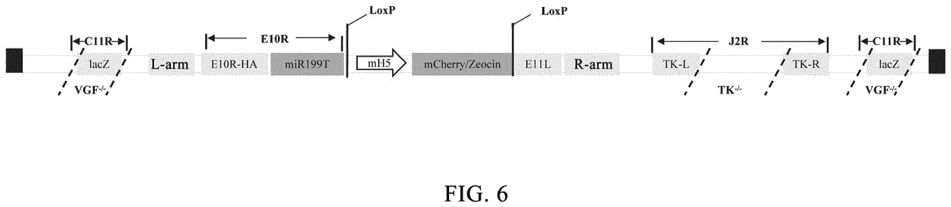

Figure 6 shows the structure of the oncolytic virus vector MiTDvv-mCherry constructed in Preparation Example 3.

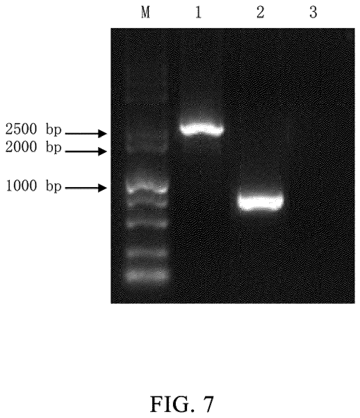

Figure 7 shows the identification results, assessed by PCR, of the monoclonal recombinant oncolytic virus of the oncolytic virus MiTDvv-hIL21-mCherry constructed in Preparation Example 4; wherein the lane M is the DNA molecular weight marker, the lane 1 is the MiTDvv-hIL21-mCherry, the lane 2 is the DDvv-hIL21, and the lane 3 is negative control (without DNA sample).

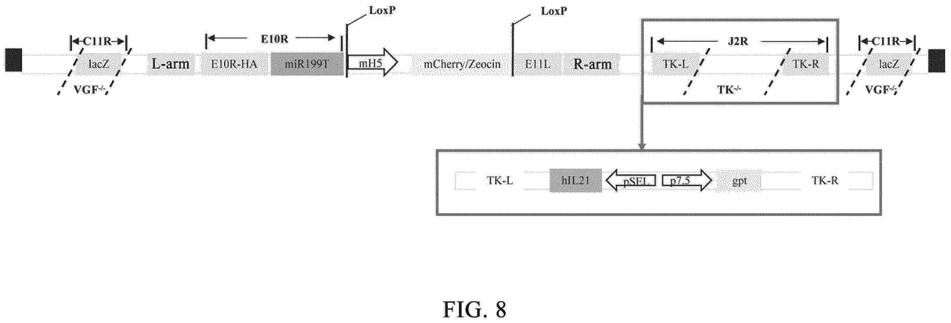

Figure 8 shows the structure of the oncolytic virus MiTDvv-hIL21-mCherry constructed in Preparation Example 4.

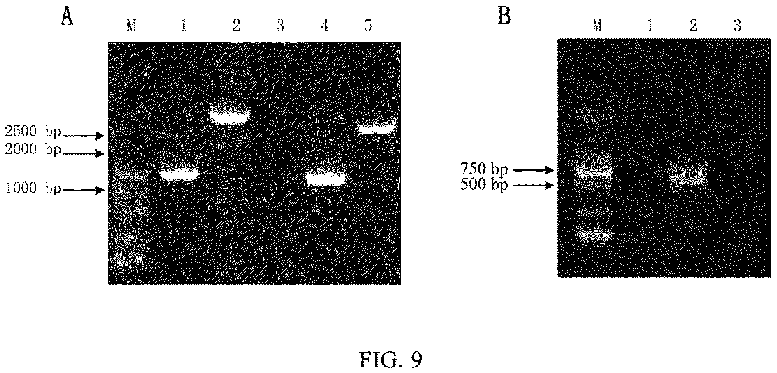

Figure 9A shows the confirmation of miR199T sequence in MiTDvv backbone virus and MiTDvv-hIL21 oncolytic virus, lacking mCherry sequence, assessed by PCR method in Preparation Example 5; wherein the lane M is the DNA molecular weight marker, the lane 1 is the MiTDvv, the lane 2 is the MiTDvv-mCherry, the lane 3 is the negative control (without DNA sample), the lane 4 is the DDvv-hIL21, and the lane 5 is the MiTDvv-hlL21-mCherry. Figure 9B shows that the MiTDvv backbone virus lacks TK gene, which was confirmed by PCR method in Preparation Example 5; wherein the lane M is the DNA molecular weight marker, the lane 1 is the MiTDvv, the lane 2 is the VSC20, and the lane 3 is the negative control (without DNA sample).

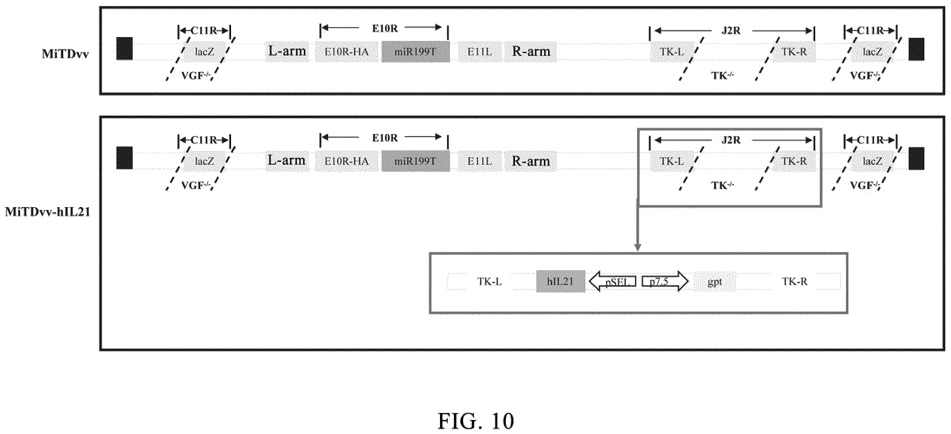

Figure 10 shows the structures of backbone virus MiTDvv and oncolytic virus MiTDvv-hIL21 constructed in Preparation Example 5.

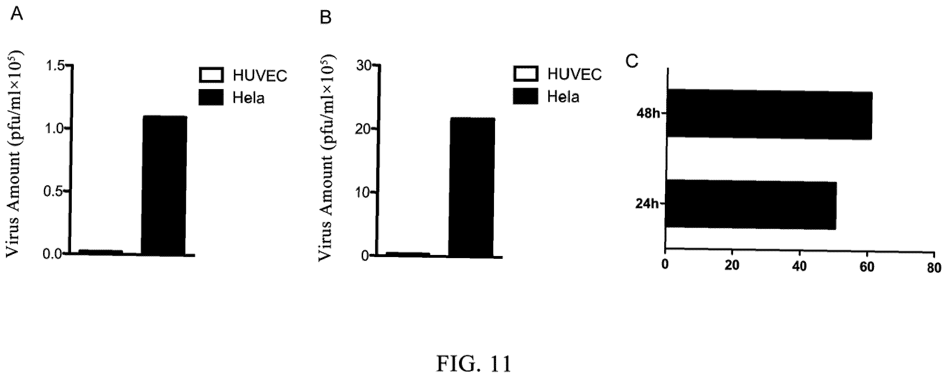

Figure 11 shows a comparison of the replication of MiTDvv backbone virus in normal cells and in tumor cells in Example 1. The results demonstrate that the replication of MiTDvv backbone virus in human normal cells HUVEC is lower than that in human tumor cells Hela, exhibiting a significant tumor selective replication function; wherein Figure 11A shows the viral amount in HUVEC cells (opened bar) and Hela cells (closed bar) after 24-hour infection with MiTDvv backbone virus, respectively; Figure 11B shows the viral amount in HUVEC cells (opened bar) and Hela cells (closed bar) after 48-hour infection with MiTDvv backbone virus, respectively; Figure 11C shows the replication fold of MiTDvv backbone virus in Hela cells relative to HUVEC cells. In Figures 11A and 11B, the X-axis represents different cells, and the Y-axis represents the viral amount per milliliter of culture medium; In Figure 11C, the X-axis represents the fold of virus replication in HeLa cells vs HUVEC cells, and the Y-axis represents the virus infection time.

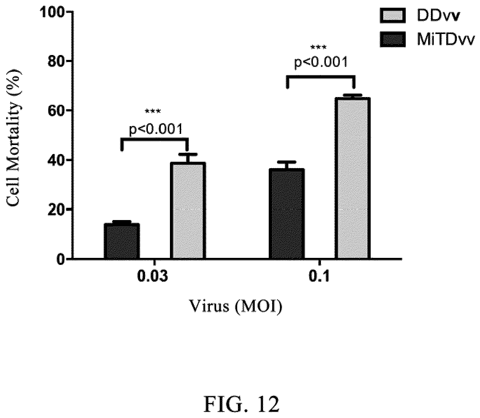

Figure 12 shows a comparison of killing effect *in vitro* of MiTDvv backbone virus and DDvv backbone virus on normal cell lines in Example 2. As shown in the figure, the killing effect of DDvv backbone virus on normal cells MRC-5 was significantly stronger than that of MiTDvv backbone oncolytic virus on normal cells MRC-5. The X-axis represents the various dose (MOI) of virus infection used to treat the cells, the Y-axis represents the corresponding percentage of killing rate, the gray bars represent DDvv virus infection, and the black bars represent MiTDvv virus infection.

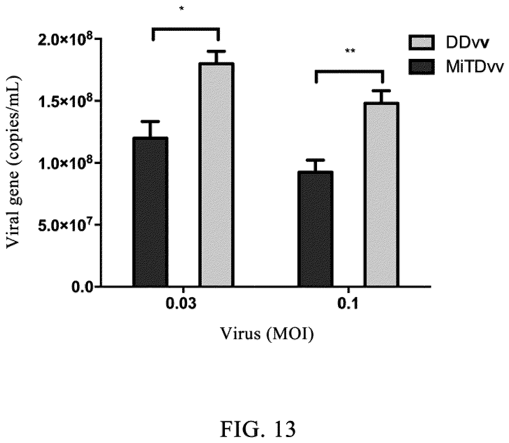

Figure 13 shows a comparison of the replication of MiTDvv backbone virus and DDvv backbone virus in normal cells in Example 3. As shown in the figure, the replication of the DDvv backbone oncolytic virus in normal cells MRC-5 was significantly higher than that of the MiTDvv backbone oncolytic virus in normal cells MRC-5. The X-axis represents the different dose (MOls) of viruses used to treat the cells, the Y-axis represents the corresponding number of copies of viral gene per milliliter of culture system, the gray bars represent DDvv virus infection, and the black bars represent MiTDvv virus infection.

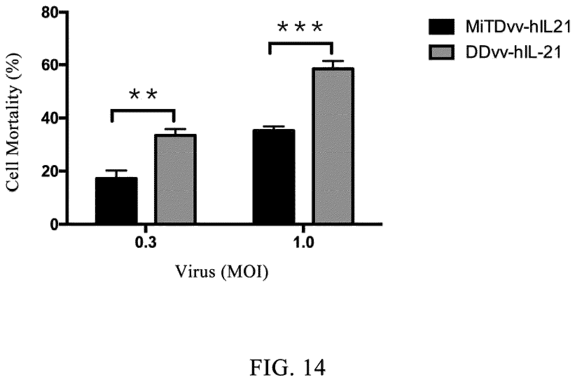

Figure 14 shows a comparison of the killing effect of oncolytic vaccinia viruses MiTDvv-hIL21 and DDvv-hIL21 on normal cells in Example 4. As shown in the figure, the killing effect of DDvv-hIL21 oncolytic virus on normal cells MRC-5 was significantly stronger than that of MiTDvv-hIL21 oncolytic virus on normal cells MRC-5. The X-axis represents the different dose (MOls) of viruses used to treat the cells, the Y-axis represents the corresponding percentage of cell growth inhibition rate, the black bars represent MiTDvv-hIL21 virus infection, and the gray bars represent DDvv-hIL21 virus infection.

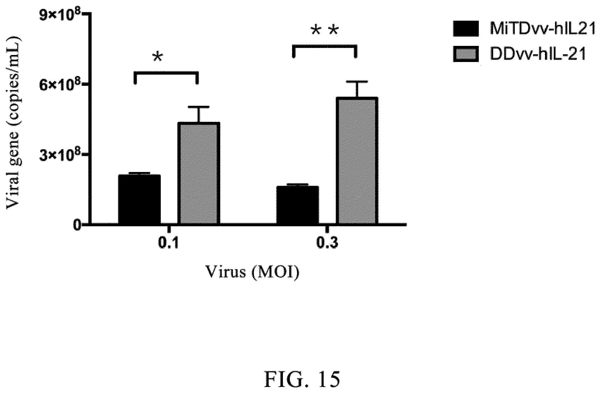

Figure 15 shows a comparison of the replication of oncolytic vaccinia viruses MiTDvv-hIL21 and DDvv-hIL21 in normal cells in Example 5. As shown in the figure, the replication of DDvv-hIL21 oncolytic viruses in normal cells MRC-5 was significantly higher than that of MiTDvv-hIL21 oncolytic viruses in normal cells MRC-5, wherein the X-axis represents the different dose (MOls) of viruses used to treat the cells, the Y-axis represents the number of copies of viral genes per milliliter of culture system, the gray bars represent DDvv-hIL21 oncolytic vaccinia virus infection, and the black bars represent MiTDvv-hIL21 oncolytic vaccinia virus infection.

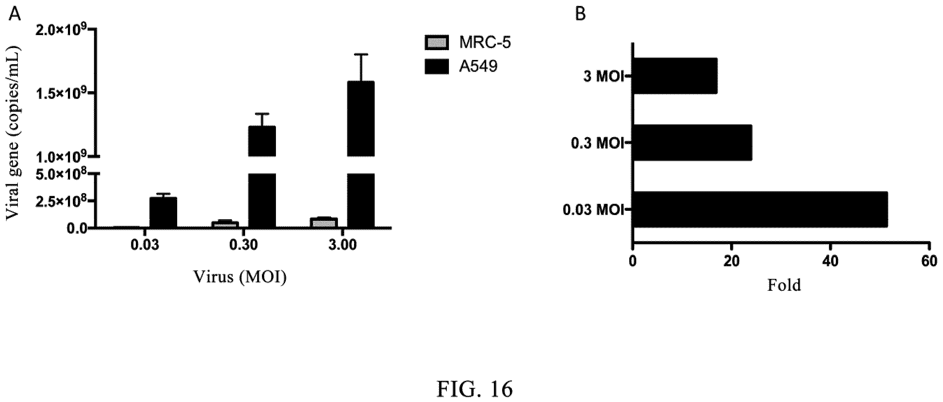

Figure 16 shows a comparison of the replication of MiTDvv-hIL21 oncolytic vaccinia virus in normal cells and in tumor cells in Example 6. As shown in the figures, the replication of MiTDvv-hIL21 oncolytic virus in normal cells MRC-5 was significantly lower than that in tumor cells A549. Figure 16A shows the viral amount in the cells infected with different titers of virus, wherein the X-axis represents the different dose (MOls) of viruses used to treat the cells, the Y-axis represents the number of copies of viral genes per milliliter of culture system, the gray bars represent that MiTDvv-hIL21 oncolytic vaccinia virus infects normal cells MRC-5, and the black bars represent that MiTDvv-hIL21 oncolytic vaccinia virus infects tumor cells A549. Figure 16B shows the replication fold of MiTDvv-hIL21 oncolytic virus in tumor cells A549 versus in normal cells MRC-5, wherein the X-axis represents the replication fold of oncolytic virus in A549 cells versus in MRC-5 cells, and Y-axis represents the different dose (MOls) of viruses used to treat cells.

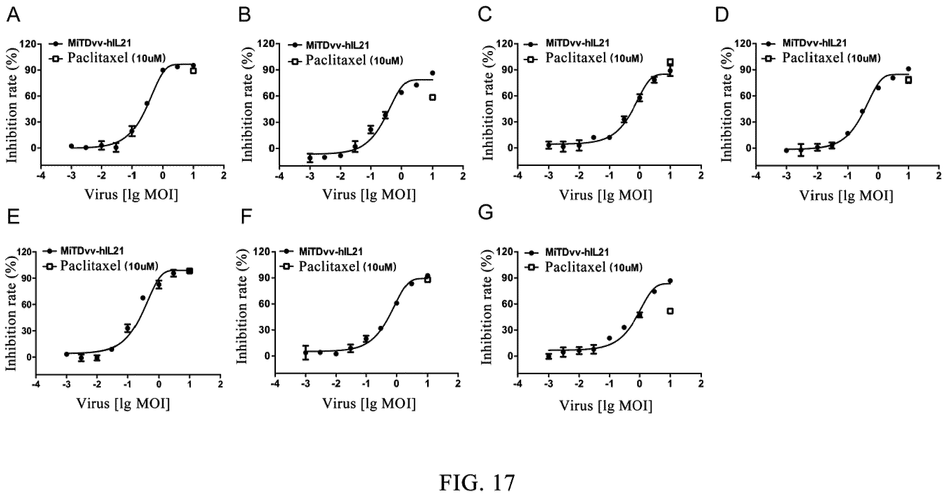

Figure 17 shows the *in vitro* killing effect of MiTDvv-hIL21 oncolytic vaccinia virus on different tumor cell lines in Example 7. Figures 17A-G represent the result of FaDu cells (A), A549 cells (B), LOVO cells (C), MNNG/HOS C1 cells (D), SK-HEP-1 cells (E), PANC-1 cells (F) and U251 cells (G), respectively. The X-axis represents the various doses [log values of MOIs] of viruses used to treat the cells, the Y-axis represents the corresponding percentage of the inhibition rate, the solid dots represents the inhibition rate of MiTDvv-hIL21 oncolytic vaccinia virus, and the opened squares represents the inhibition rate of positive control of 10 µM paclitaxel.

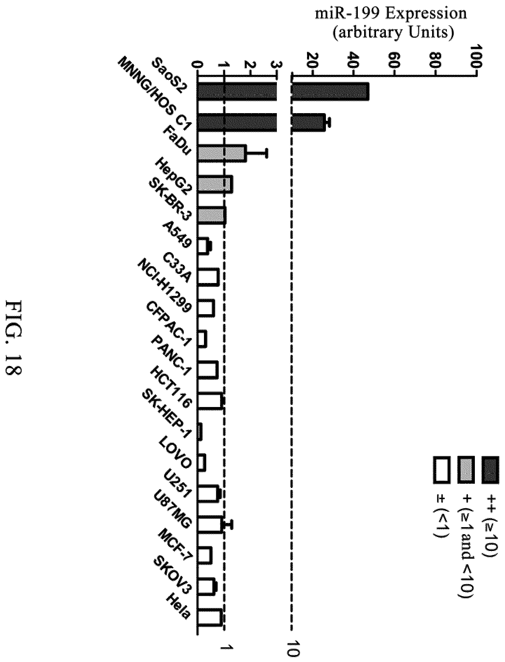

Figure 18 shows the expression levels of miR-199 in different cells in Example 8, wherein the X-axis represents the various tumor cell lines, and the Y-axis represents the relative expression level of miR-199. Black bars represent high-expressing cell lines, gray bars represent medium-expressing cell lines, and white bars represent low-expressing cell lines.

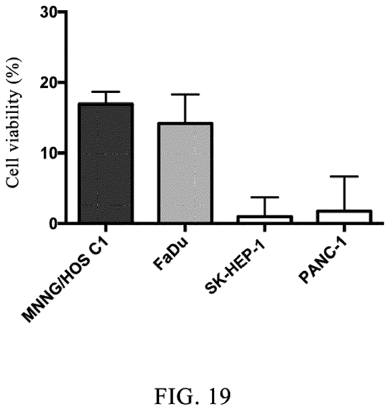

Figure 19 shows the correlation between the killing effect of MiTDvv-hIL21 oncolytic virus on tumor cells and the expression levels of miR-199 in Example 8, wherein the X-axis represents the different tumor cell lines, and the Y-axis represents the percentage of cell viability.

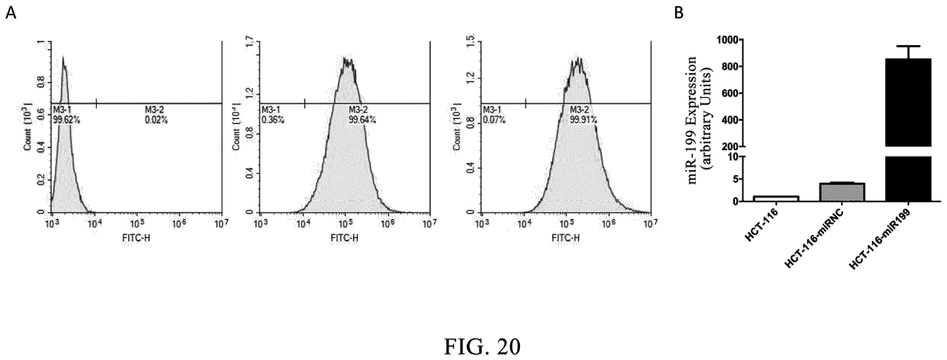

Figure 20 shows the verification of the stable expression of miR-199 fragments in HCT116 cells in Example 9. Figure 20A shows the results of detecting GFP expression by flow cytometry, in which the left panel is the result of a blank control group (HCT116 cell line), the middle panel is the result of a negative control cell line HCT116-miRNC, and the right panel is the result of the HCT116-miR199 cell line which stably expresses miR-199; Figure 20B shows the results of the expression of miR-199 by quantitative PCR detection, in which the X axis represents the different cell lines, and the Y axis represents the relative expression level of miR-199.

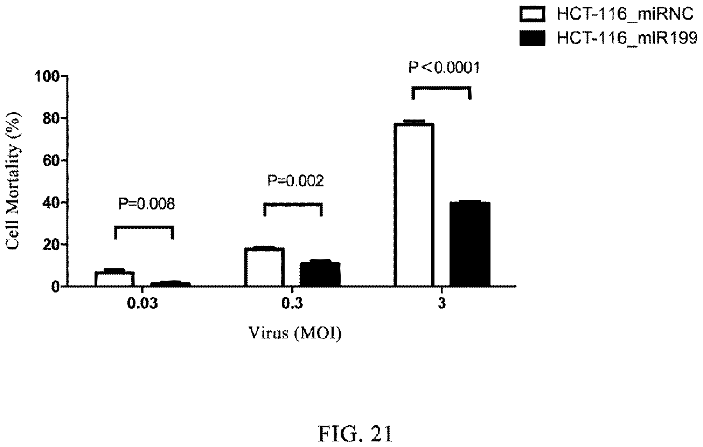

Figure 21 shows the results of the infection of tumor cells high-expressing miR-199 with MiTDvv-hIL21 oncolytic virus in Example 9. As shown in the figure, the tumor cells high-expressing miR-199 significantly inhibited the cell killing effect of MiTDvv-hlL21 oncolytic virus. The X-axis represents the different doses (MOls) of viruses, the Y-axis represents the percentage of the corresponding cell growth inhibition rate, the white bars represent the cell line HCT116-miRNC, and the black bars represent the cell line HCT116-miR199.

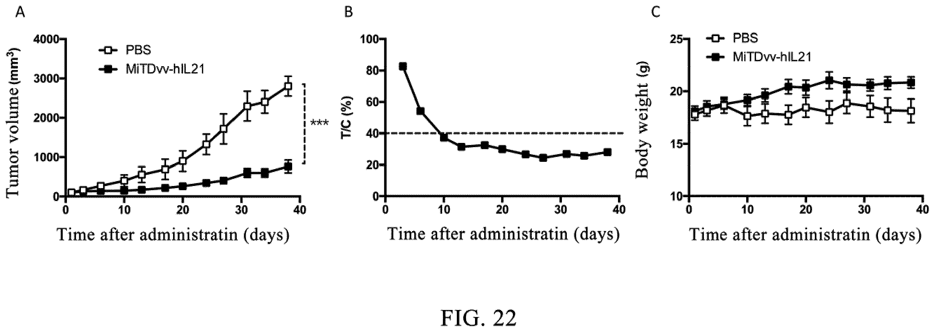

Figure 22 shows the tumor suppression effect of MiTDvv-hIL21 oncolytic virus on colorectal cancer in Example 10. Figure 22A shows the time-course changes of tumor volume after intratumoral injection of MiTDvv-hIL21 oncolytic vaccinia virus. The figure shows that MiTDvv-hIL21 oncolytic vaccinia virus can effectively inhibit the growth of tumor; wherein the X-axis represents the time after administration, Y-axis represents tumor volume, solid squares indicate intratumoral injection of MiTDvv-hIL21, and opened squares indicate intratumoral injection of PBS. Figure 22B shows the time-course changes of relative tumor growth rate T/C (%) after intratumoral injection of MiTDvv-hIL21 oncolytic vaccinia virus. As shown in the figure, the relative tumor growth rate of the MiTDvv-hIL21-administered group was under 40% from Day 10 after the administration; wherein the X-axis represents the time after the administration, the Y-axis represents T/C (%), and the solid squares indicate intratumoral injection of MiTDvv-hIL21. Figure 22C shows the time-course changes of mice body weight after intratumoral injection of MiTDvv-hIL21 oncolytic vaccinia virus. As shown in the figure, there was no significant decrease in the body weight of mice throughout the experiment; wherein the X axis represents the time after administration, the Y axis represents the body weight of mice, solid bars indicate intratumoral injection of MiTDvv-hlL21, and opened squares indicate intratumoral injection of PBS.

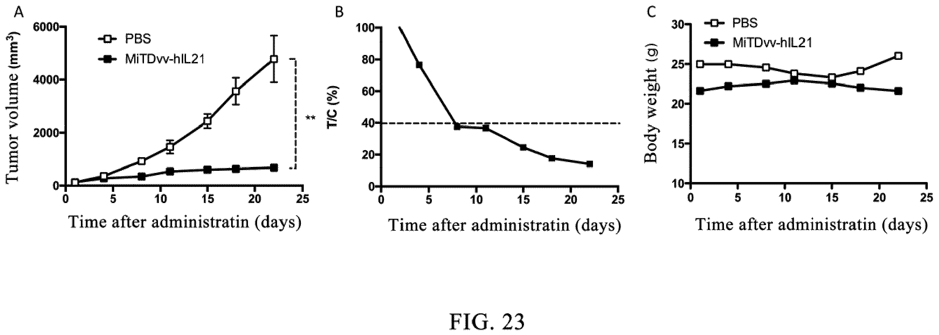

Figure 23 shows the tumor suppression effect of MiTDvv-hIL21 oncolytic virus on human osteosarcoma in Example 11. Figure 23A shows the time-course changes of tumor volume after intratumoral injection of MiTDvv-hIL21 oncolytic vaccinia virus. The figure shows that MiTDvv-hIL21 oncolytic vaccinia virus can effectively inhibit tumor growth; wherein the X axis represents the time after administration, Y-axis represents tumor volume, solid squares indicate intratumoral injection of MiTDvv-hlL21, and opened squares indicate intratumoral injection of PBS. Figure 23B shows the time-course changes of the relative tumor growth rate T/C (%) after intratumoral injection of MiTDvv-hIL21 oncolytic vaccinia virus. The figure shows that the relative tumor growth rate of MiTDvv-hIL21 administration group was less than 40% from Day 8 after the administration; the X-axis represents the time after administration, the Y-axis represents T/C (%), and the solid squares indicate intratumoral injection of MiTDvv-hIL21. Figure 23C shows the change in the body weight of mice over time after intratumoral injection of MiTDvv-hIL21 oncolytic vaccinia virus. As shown in the figure, there was no significant decrease in the body weight of mice throughout the experiment; wherein the X axis represents the time after administration, the Y axis represents the body weight of mice, solid squares indicate intratumoral injection of MiTDvv-hl L21, and hollow squares indicate intratumoral injection of PBS.

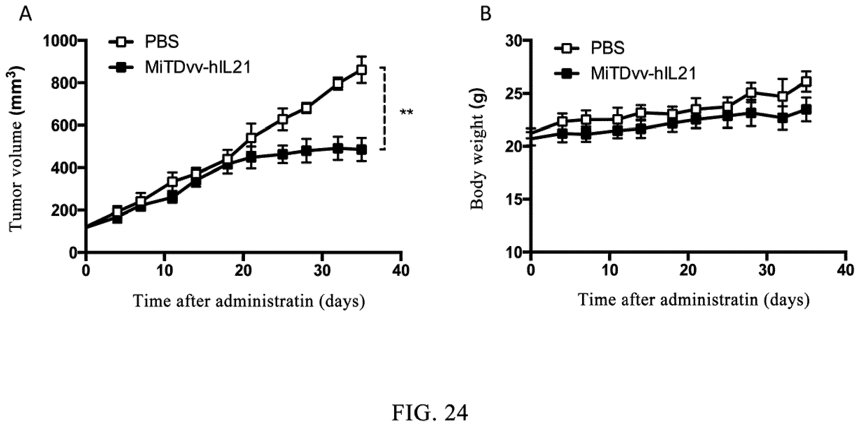

Figure 24 shows the tumor suppression effect of MiTDvv-hIL21 oncolytic virus on human liver cancer in Example 12. Figure 24A shows the time-course changes of tumor volume after intratumoral injection of MiTDvv-hIL21 oncolytic vaccinia virus. It can be seen from the figure that MiTDvv-hIL21 oncolytic vaccinia virus can effectively inhibit tumor growth; wherein the X-axis represents the time after administration, Y-axis represents the tumor volume, solid squares indicate intratumoral injection of MiTDvv-hIL21, and opened squares indicate intratumoral injection of PBS. Figure 24B shows the time-course changes in the body weight of mice after intratumoral injection of MiTDvv-hIL21 oncolytic vaccinia virus. As shown in the figure, there was no significant decrease in the body weight of mice throughout the experiment; wherein the X axis represents the time after administration, the Y axis represents the body weight of mice, solid squares indicate intratumoral injection of MiTDvv-hIL21, and opened squares indicate intratumoral injection of PBS.

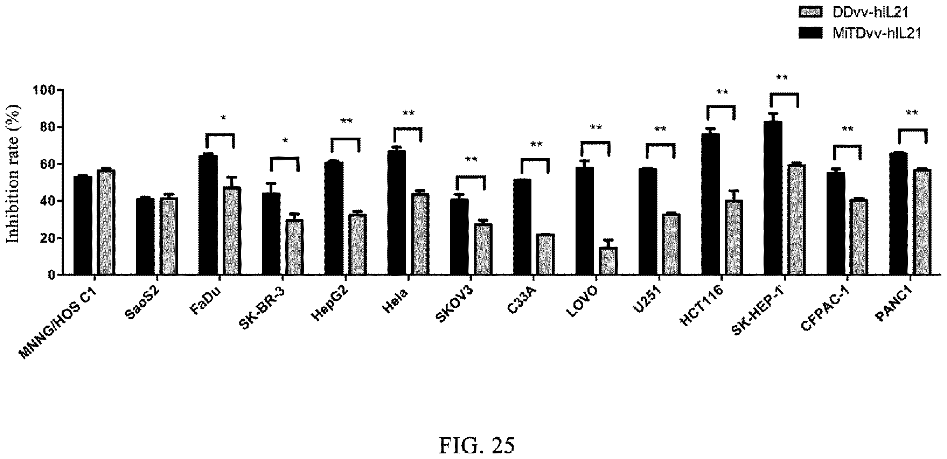

Figure 25 shows the evaluation of killing effect of oncolytic vaccinia viruses MiTDvv-hIL21 and DDvv-hIL21 on tumor cells with different miR-199 expression levels in Example 13, wherein the X-axis represents the different types of tumor cells, the Y-axis represents the cell growth inhibition rate, the black bars represent the MiTDvv-hIL21 oncolytic vaccinia virus, and the gray bars represent the DDvv-hIL21 oncolytic vaccinia virus.

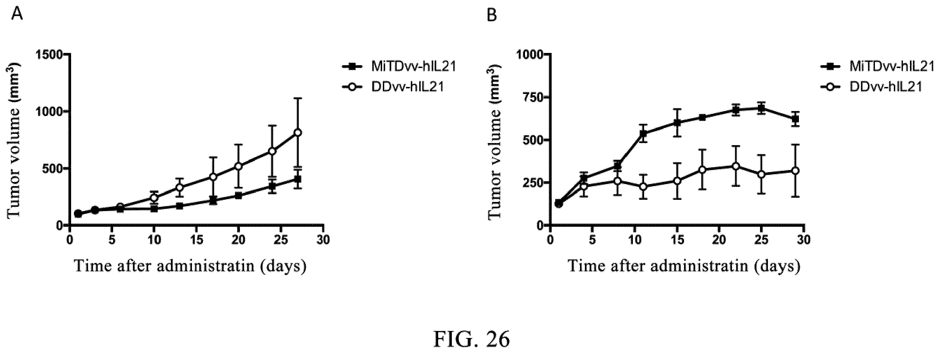

Figure 26 shows a comparison of tumor suppression effects of oncolytic vaccinia viruses MiTDvv-hIL21 and DDvv-hIL21 on tumor-bearing mice with different miR-199 expression levels in Example 14. Figure 26A shows the time-course changes of tumor volume after intratumoral injection of oncolytic vaccinia virus in tumor-bearing mice of human colorectal cancer HCT116 cells, which is low-expressing miR-199; wherein the X axis represents the time after administration, the Y-axis represents the tumor volume, solid squares indicate intratumoral injection of MiTDvv-hIL21, and opened squares indicate intratumoral injection of DDvv-hIL21. The figure shows that MiTDvv-hIL21 has stronger tumor suppression effect than DDvv-hIL21. Figure 26B shows the time-course changes of tumor volume after intratumoral injection of oncolytic vaccinia viruses in tumor-bearing mice of human osteosarcoma MNNG/HOS C1 cells, which is high-expressing miR-199; wherein the X axis represents the time after administration, the Y-axis represents the tumor volume, solid squares represent the intratumoral injection of MiTDvv-hIL21, and opened squares represent the intratumoral injection of DDvv-hIL21. The figure shows that MiTDvv-hIL21 has a weaker tumor suppression effect than DDvv-hIL21.

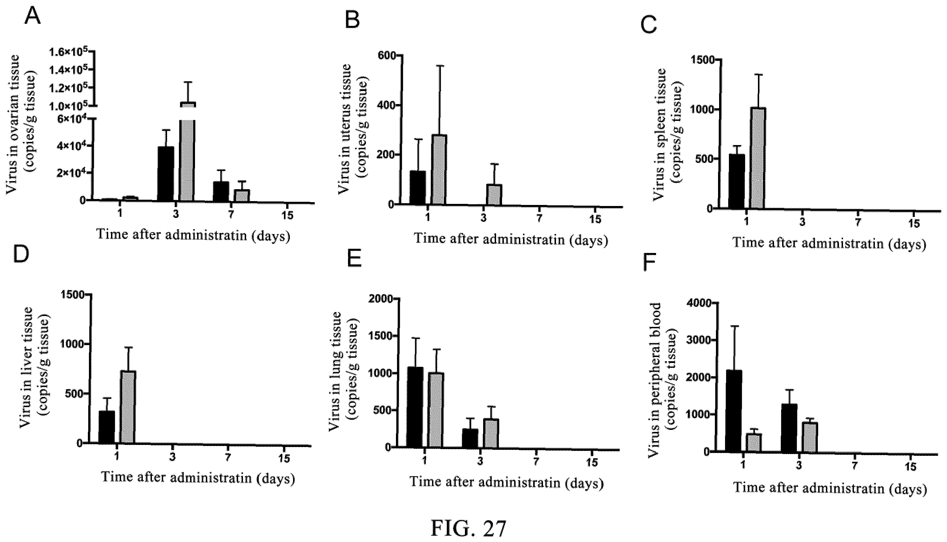

Figure 27 shows the tissue distribution of oncolytic vaccinia viruses MiTDvv-hIL21 and DDvv-hIL21 in mice in Example 15. Figures 27A-F show the distribution of oncolytic vaccinia viruses MiTDvv-hIL21 and DDvv-hIL21 in ovary, uterus, spleen, liver, lung, peripheral blood of mice, respectively. The X-axis represents the time after administration, the Y-axis represents the viral amount in the tissues, the black bars represent MiTDvv-hIL21 oncolytic vaccinia viruses, and the gray bars represent DDvv-hlL21 oncolytic vaccinia viruses.

In Figures 12-15 and 22-25, "*" means p<0.05, "**" means p<0.01, and "***" means p<0.001 (compared with the corresponding control group, obtained by One-way ANOVA statistical analysis method).

### Detailed Description of the Preferred Embodiments

The present invention is further explained with the following detailed description of preferred embodiments with references to the accompanying drawings, which is not to be taken in a limiting sense, and it will be apparent to those skilled in the art that various modifications or improvements can be made accordingly without departing from the spirit of the present invention and these are therefore within the scope of the present invention.

In the present invention, the words "tumor", "cancer", "tumor cell", and "cancer cell" encompass a meaning commonly recognized in the art.

As used herein, the term "oncolytic virus" or "oncolytic vaccinia virus" refers to a virus or a vaccinia virus that can replicate selectively in tumor cells and lyse tumor cells.

As used herein, the term "therapeutically effective dose" refers to an amount of a functional agent or of a pharmaceutical composition useful for exhibiting a detectable therapeutic or inhibitory effect or invoking an antitumor effect. The effect can be detected by any assay method known in the art.

As used herein, the term "administer" or "administration" refers to providing a compound, a composite or a composition (including viruses and cells) to a subject.

As used herein, the term "patient" refers to a human or non-human organism. Thus, the methods and compositions described herein are applicable to both human and veterinary diseases. In certain embodiments, the patient has a tumor. In some cases, the patient may suffer from one or more types of cancer simultaneously.

As used herein, the term "synergistic effect" refers to an effect arising between two or more agents that produce an effect greater than the sum of their individual effects.

As used herein, the term "pfu", or "plaque forming unit" refers to the number of viruses forming a plaque.

As used herein, the term "MOI", or "multiplicity of infection" refers to the ratio between the number of viruses and the number of cells, i.e., the number of virus particles used to initiate viral infection per cell. MOI = pfu/cell, that is, the number of cells × MOI = Total PFU.

The strategy based on the use of endogenous microRNAs (miRNAs) to control the viral replication have been widely used (see the reference "Ylösmäki et al., Generation of a conditionally replicating adenovirus based on targeted destruction of E1A mRNA by a cell type-specific MicroRNA. J Virol 82: 11009-11015. 2008"). miRNA is a 20-24bp of small nucleic acid molecule that plays an important biological role in the expression of target genes or the modification of post-translational protein by binding to the non-coding sequence of a specific target gene (see the reference "Ambros et al., The functions of animal microRNAs. Nature 431: 350-355 2004"). In cancer researches, it was found that the expression of a series of miRNA small molecules changed significantly during tumor progression (see the reference " Negrini et al., MicroRNAs in human cancer: from research to therapy. J Cell Sci 120: 1833-1840.2007"), exhibiting multi-target regulation of cancer-related genes, such as mTOR, c-Met, HIF-1α, and CD44 etc. (see the references "Fornari et al., miR-199a-3p regulates mTOR and c-Met to influence the doxorubicin sensitivity of human hepatocarcinoma cells. Cancer Res 70: 5184-5193. 2010"; "Kim et al., MicroRNA miR-199a regulates the MET proto-oncogene and the downstream extracellular signal-regulated kinase 2 (ERK2). J Biol Chem 283:18158 -18166.2008"; "Jia et al., Lentivirus-Mediated Overexpression of MicroRNA-199a Inhibits Cell Proliferation of Human Hepatocellular Carcinoma. Cell Biochem Biophys, 62: 237-44.2011"; "Henry et al., miR-199a-3p targets CD44 and reduces proliferation of CD44 positive hepatocellular carcinoma cell lines. Biochem Biophys Res Commun 403: 120-125.2010").

In order to provide a novel recombinant oncolytic vaccinia virus with significant selectivity for tumor cells, the inventors have selected a specific essential gene E10R from the genome of the oncolytic vaccinia virus through intensive research and experimental exploration, and proposed to insert an exogenous nucleotide sequence into 3'UTR region (3' untranslated region) of the E10R, wherein the exogenous nucleotide sequence contains a target sequence of a specific microRNA which has a lower expression level in tumor cells than in normal cells. In this way, in normal cells infected with the oncolytic vaccinia virus, the high-expressing microRNA can target the corresponding mRNA in the 3'UTR region of E10R of the oncolytic vaccinia virus and inhibit the expression of E10R by degrading the mRNA or hindering its translation, thereby inhibiting the replication of the oncolytic vaccinia virus. In contrast, in tumor cells infected with the oncolytic vaccinia virus, the microRNA is low-expressed or not expressed, so that the expression of E10R is not inhibited, thereby maintaining the replication ability of the oncolytic vaccinia virus. Therefore, by utilizing the characteristic that the expression level of specific microRNA in normal tissues may be different than that in tumor tissues *in vivo,* the present invention provides a novel recombinant oncolytic vaccinia virus with higher and more significant selectivity to tumor cells. The inventors have surprisingly found that the recombinant oncolytic vaccinia virus of the present invention has a significantly higher replication in various tumor cells than in normal cells, thereby providing superior tumor-target specificity and safety, and can significantly inhibit the growth of tumor cells. Furthermore, since the novel recombinant oncolytic vaccinia virus can only replicate in tumor cells lacking specific microRNAs, it can achieve a stronger tumor cell selectivity and reduce the effect of the viruses on normal tissues.

Based on the above concept, the present invention provides an isolated recombinant oncolytic vaccinia virus capable of being regulated by a microRNA which has lower expression level in tumor cells of a mammal than in normal cells of the same mammal, wherein the 3'UTR region of the E10R gene in the recombinant oncolytic vaccinia virus genome is integrated with a target sequence of the microRNA.

The microRNA has a lower expression level in tumor cells of a mammal than in normal cells of the same mammal, which means that the expression level of the microRNA in at least one kind of tumor cell of the mammal is lower than that in at least one kind of normal cell of the same mammal. The mammal include human.

It is found in the present invention that the replication of the recombinant oncolytic vaccinia virus in tumor cells is significantly higher than that in normal cells, and the growth of tumor cells can be significantly inhibited.

The microRNAs include those with low-expression or no expression in tumor cells known in the art, and also include those with low-expression or no expression in tumor cells determined by techniques known in the art. The microRNA may be selected from a group consisting of miR-9, miR-15a, miR-16, miR-26a, miR-27b, miR-29b, miR-30a, miR-32, miR-33, miR-34, miR-95, miR-101, miR-122, miR-124, miR-125a, miR-125b, miR-126, miR-127, miR-128, miR-133b, miR-139, miR-140, miR-142, miR-143, miR-145, miR-181, miR-192, miR-195, miR-198, miR-199a, miR-199b, miR-200, miR-203, miR-204, miR-205, miR-218, miR-219, miR-220, miR-224, miR-345 and miR-375; preferably miR-199a and miR-199b. Among them, miR-199 (including miR-199a and miR-199b) can most effectively achieve the purpose of the present invention and the resulting recombinant oncolytic vaccinia virus shows stronger tumor selectivity and safety. In the present invention, a miRNA has a concept that includes any one of a miRNA precursor and a mature miRNA, but, unless otherwise specified, it refers to a mature miRNA.

In the present specification, the names and the numbers of the above-mentioned microRNAs are recognized and well-known in the art (for example, the mature miRNA is abbreviated as miR and the highly homologous miRNAs have their numbers followed by English lowercase letters, etc., such as miR-199a and miR-199b), which respectively have known specific nucleotide sequences, and the specific nucleotide sequences can be searched in the miRBase public database website (http://www.mirbase.org/).

Mature miRNAs (mature RNAs) or miRNA mature forms are produced by the cleavage of longer primary transcripts by a series of nucleases. Primary transcripts (pri-miRNAs) vary in length from hundreds to thousands of bases, with a cap at 5' end and a polyA tail at 3' end, as well as one to several hairpin stem-loop structures. The primary transcript is cleaved to produce a precursor miRNA of about 70 bases, that is, a pre-miRNA. The precursor miRNA (pre-miRNA) is further cleaved to form a single-stranded mature miRNA of about 22 bases in length. In cells, pri-miRNAs and pre-miRNAs as well as mature miRNAs may be present in general. In the present specification, miRNA has a concept including either a precursor miRNA or a mature miRNA, but, unless otherwise specified, it refers to a mature miRNA.

Preferably, the target sequence of the microRNA is repeated, which preferably contains 2-8 repeats, more preferably 3-4 repeats. In some embodiments, the repeated target sequences of the microRNA are separated by a spacer with 2 or more nucleotides (e.g., gg, cc and ggcc). The repeated target sequence of the miRNA can be a reverse complement of the miRNA.

Preferably, the recombinant oncolytic vaccinia virus is TK gene functionally deficient and/or VGF gene functionally deficient. The oncolytic vaccinia virus thus constructed achieves enhanced tumor cell selectivity and has significantly reduced replication ability in and killing effect on normal cells, compared with known oncolytic vaccinia virus (such as TK/VGF double-deficient vaccinia virus).

The term "functionally deficient" used in the present invention when referring to the gene of an oncolytic virus means that the oncolytic virus cannot perform the function that the gene should have inherently, that is, the function is lost. This purpose can be achieved by, for example, inserting an exogenous fragment into the gene or knockout of the gene.

Therefore, an exogenous nucleotide sequence can be inserted into the TK gene to make it functionally deficient. It is also possible to insert an exogenous nucleotide sequence into the VGF gene and/or to knock out the VGF gene to make it functionally deficient.

Preferably, an exogenous IL-21 gene is integrated into the genome of the recombinant oncolytic vaccinia virus, and the IL-21 gene can be expressed in tumor cells.

IL-21 (i.e., interleukin-21) is a multi-directional type I cytokine, mainly produced by T cells, which regulates innate and acquired immune responses, and plays an important role in the anti-tumor immune response. The effects of IL-21 on various immune cells and signal transduction have been reported in a literature (see the reference: "Leonard, WJ & Wan, C. IL-21 Signaling in Immunity. F1000Research 5, 1-10 (2016)"). Various types of immune cells mainly include: 1) CD4⁺ T cells: promote proliferation and produce cytokines; T_{fh} cells: promote differentiation and improve development center function; Tₕ₁₇ cells: promote differentiation and proliferation; T_{reg} cells: inhibit production and survival; 2) NKT cells: proliferate and enhance cytotoxicity; 3) CD8⁺ T cells: improve cytotoxicity, proliferation and/or survival, anti-tumor effect; 4) NK cells: promote cell maturation, proliferation, increase cytotoxic effect, and enhance anti-tumor activity; 5) DC cells: inhibit antigen presentation function and induce apoptosis; 6) macrophages: enhance phagocytosis; 7) B cells: promote proliferation and/or apoptosis, promote plasma cell differentiation and immunoglobulin production; 8) In addition, IL-21 can activate a variety of tumor-related signal channels, including JAK/STAT, MARK/PI3K and other signal channels, and regulate the progression of tumors. In tumor immunotherapy, activation of cytotoxicity of NK cells and CD8⁺ T cells is the key, and many studies have fully shown that IL-21 plays an important role in this procedure. IL-21 promotes maturation of NK cells to produce IFN-γ, granzyme B and perforin, and induces NK cell-mediated anti-tumor cytotoxicity, and increasing the lethality of NK cells via the antibody-dependent cell-mediated cytotoxicity (ADCC) (see the reference: "Spolski, R. & Leonard, WJ Interleukin-21: a double-edged sword with therapeutic potential. Nat. Rev. Drug Discov. 13, 379-395, 2014."). Secondly, IL-21 can induce the proliferation of CD8⁺ T cells, induce the generation of memory T cells and promote the secretion of IFNγ/granzyme B to enhance the killing of tumors by CD8 ⁺ T cells and contribute to the memory immune response to recurrent tumor cells. Importantly, unlike IL-2, IL-21 does not induce the expansion of T_{reg} cells, and further enhances the immune function response of CD8⁺T cells (see the reference: "Spolski, R. & Leonard, WJ Interleukin-21: a double-edged sword with therapeutic potential. Nat. Rev. Drug Discov. 13, 379-395, 2014." Based on the diverse effects of IL-21 on immune cells, it is shown that IL-21 can "reactivate" a variety of effector cells in the tumor microenvironment.

The invention further strengthens the anti-tumor effect of the novel oncolytic vaccinia virus by inserting the IL-21 gene, which is capable of regulating immunity, into the genome of the oncolytic vaccinia virus. In this way, the oncolytic virus can selectively replicate in tumor cells and kill the tumor cells and further induce the subsequent immune response of the body, and meanwhile, the anti-tumor immune effect of exogenous IL-21 can be fully exerted. The present invention found that integrating the IL-21 gene into the oncolytic vaccinia virus can produce a synergistic effect on the oncolytic killing effect of the oncolytic virus and the anti-tumor immune stimulating effect of IL-21.

Preferably, the exogenous IL-21 gene is inserted into the TK locus, so that the TK gene can be functionally deficient and the IL-21 gene can be expressed after the infection of tumor cells.

Vaccinia viruses that can be used in the present invention include a Wyeth strain or a WR strain, and an example of the WR strain is VSC20.

In a preferred embodiment, the recombinant oncolytic vaccinia virus is obtained by genetically modifying the VSC20 vaccinia virus, a vaccinia virus lack of VGF gene in which the LacZ gene is inserted at the C11R locus. The preparation method thereof can be found in the scientific literature: "McCart, JA, et al. Systemic cancer therapy with a tumor-selective vaccinia virus mutant lacking thymidine kinase and vaccinia growth factor genes. Cancer Res (2001) 61: 8751-8757". The genetic modification includes inserting a repeated target sequence of specific microRNA, such as a target sequence of miR-199 (miR-199a-3p or miR-199b-3p), in 3'UTR region of E10R gene of the VSC20 vaccinia virus.

In some embodiments, the present invention utilizes the means of genetic engineering to construct a shuttle plasmid containing the left and right homologous arms of the E10R gene and repeated target sequence of specific microRNAs, and then introduces the shuttle plasmid into the VSC20 vaccinia virus. A recombinant virus with the repeated target sequence of specific microRNAs inserted into 3'UTR region of E10R gene was obtained through a recombination mechanism. The flow chart of a specific embodiment of the construction of the shuttle plasmid is shown in Figure 1.

The genetic modification can also include inserting an exogenous sequence (such as the IL-21 gene) into the TK gene of the VSC20 vaccinia virus, thereby making the TK gene functionally deficient. The specific method of which is as described in Preparation Example 3 or 4, or can be as described in Chinese Patent Publication No.CN109554353A, the entire contents of which are incorporated herein by reference.

The genome of the recombinant oncolytic vaccinia virus may also integrate with an exogenous screening gene. The exogenous screening gene includes gpt (guanine phosphoribosyl transferase) gene, LacZ gene and fluorescent protein gene (such as red fluorescent protein gene). Preferably, it does not include fluorescent protein gene to avoid the potential safety hazards of fluorescent protein expressed in patients.

The genome of the recombinant oncolytic vaccinia virus may not be integrated with an exogenous screening gene either.

In some embodiments, the present invention controls the gpt gene by using the vaccinia virus promoter p7.5, and controls the exogenous IL-21 gene by using the synthetic vaccinia virus early/late promoter pSEL, respectively, wherein the gpt and IL-21 genes were inserted into the TK gene region of the vaccinia virus VSC20 strain by using *in vitro* intracellular recombination technology to construct the oncolytic virus. The two promoters respectively activate the expression of the respective regulated genes in a back-to-back manner.

Preferably, the exogenous IL-21 gene is derived from mouse or human.

The recombinant oncolytic vaccinia viruses of the present invention may be obtained by related known methods in the field of bioengineering.

Based on the recombinant oncolytic vaccinia virus developed by the present invention, the present invention also provides a pharmaceutical composition, wherein the pharmaceutical composition comprises the recombinant oncolytic vaccinia virus according to the present invention as an active ingredient, and a pharmaceutically acceptable excipient.

Preferably, the pharmaceutical composition comprises a therapeutically effective amount of the recombinant oncolytic vaccinia virus. In certain embodiments, the active ingredient of the pharmaceutical composition comprises the recombinant oncolytic vaccinia virus according to the present invention at a dose of 1 × 10⁵ to 5 × 10⁹ pfu/day (e.g., 1 × 10⁵ to 3 × 10⁹ pfu/day, 1 × 10⁵ to 1 × 10⁸ pfu/day, etc.).

The oncolytic virus may be administered by the administration methods commonly used in the art, such as intratumoral injection or intravenous administration.

The pharmaceutical composition of the present invention may also contain other active ingredients known in the art, such as interleukin-2 (IL-2), IL-15, IL-17, IL-18, granulocyte-macrophage colony-stimulating factor (GM-CSF), interferon-γ (IFN-γ), tumor necrosis factor-α (TNF-α), and the like, and the dosage and administration route thereof may be performed in their respective conventional manners. If other active ingredients are included, the recombinant oncolytic vaccinia virus should be present in the pharmaceutical composition independently without being mixed with other active ingredients. For example, the recombinant oncolytic vaccinia virus is individually packaged in a separate container.

Those skilled in the art will understand that the pharmaceutical composition of the present invention may further comprise suitable pharmaceutically acceptable excipients.

In some embodiments, a pharmaceutical composition of the invention comprises one or more pharmaceutically acceptable carriers. Pharmaceutical formulations may be prepared by procedures known in the art. For example, the active ingredients including compounds and the like may be formulated with common excipients, diluents (such as phosphate buffer or saline), tissue-culture medium, and carriers (such as autologous plasma or human serum albumin) and administered as a suspension. Other carriers can include liposomes, micelles, nanocapsules, polymeric nanoparticles, solid lipid particles (see, e.g., E. Koren and V. Torchilin, Life, 63:586-595, 2011). Details on techniques for formulation of the pharmaceutical composition disclosed herein are well described in the scientific and patent literature, see, e.g., the latest edition of Remington's Pharmaceutical Sciences, Maack Publishing Co., Easton PA ("Remington's").

Another aspect of the present invention also provides a vector for preparing the recombinant oncolytic vaccinia virus according to the present invention.

The vector can modify E10R gene in the vaccinia virus through a recombination mechanism. For example, in a specific embodiment, as shown in Figure 4, the recombinant vector comprises the left and right homologous arms of the E10R gene, the repeated target sequence of miR-199 (hsa-miR-199a-3p or hsa-miR-199b-3p) and an expression frame initiating the expression of the exogenous screening gene mCherry/Zeocin. Preferably, the vector can cause TK gene functionally deficient in the vaccinia virus through a recombination mechanism. In another specific embodiment, as shown in Figure 6, the recombinant vector comprises the left and right homologous arms of the E10R gene, the repeated target sequence of miR-199 (hsa-miR-199a-3p or hsa-miR-199b-3p), an expression frame initiating the expression of the exogenous screening gene mCherry/Zeocin and the TK homologous fragments TK-L and TK-R. When the vaccinia virus is a WR strain, the sequence of the TK gene is the sequence shown in 80724-81257bp of the vaccinia virus gene numbered NC_006998 in the GenBank of the NCBI (i.e., the National Center for Biotechnology Information, URL: https://www.ncbi.nlm.nih.gov), the sequence of TK-L may be, for example, the sequence fragment shown in 80724-80961bp, and the sequence of TK-R may be, for example, the sequence fragment shown in 81033-81257bp. The insertion site of the repeated target sequence of miR-199 (hsa-miR-199a-3p or hsa-miR-199b-3p) may be, for example, at 56974 bp of the left homologous arm of the E10R gene. In another specific embodiment, as shown in Figure 8, the recombinant vector comprises the left and right homologous arms of the E10R gene, the repeated target sequence of miR-199 (hsa-miR-199a-3p or hsa-miR-199b-3p), an expression frame initiating the expression of the exogenous screening gene mCherry/Zeocin, the TK homologous fragments TK-L, TK-R, and an expression frame initiating the expressions of IL-21 gene and exogenous screening gene gpt. The vector can insert the IL-21 gene expression frame and the gpt gene expression frame into the TK gene region of the vaccinia virus through the intracellular recombination mechanism (for example, the sequence fragment shown by the 80962-81032bp in the vaccinia virus gene numbered NC_006998 in GenBank is deleted), so that the recombinant vaccinia virus loses the function of the TK gene. The insertion site of the repeated target sequence of miR-199 (hsa-miR-199a-3p or hsa-miR-199b-3p) may be, for example, at 56974 bp of the left homologous arm of the E10R gene.

Another aspect of the present invention also provides a host cell containing the vector of the present invention.

Another aspect of the present invention also provides the use of the recombinant oncolytic vaccinia virus according to the present invention in the preparation of drugs for treating tumors and/or cancers.

The tumors and/or cancers include, but are not limited to, lung cancer, melanoma, head and neck cancer, liver cancer, intracranial tumors (such as gliomas), colorectal cancer, bladder cancer, breast cancer, ovarian cancer, uterine cancer, cervical cancer, gastric cancer, esophageal cancer, kidney cancer, prostate cancer, pancreatic cancer, lymphatic cancer, leukemia, bone cancer, testicular cancer and osteosarcoma.

Another aspect of the present invention also provides a method for treating tumors and/or cancers, comprising administering to a tumor and/or cancer patient the recombinant oncolytic vaccinia virus according to the present invention.

The tumors and/or cancers include, but are not limited to, lung cancer, melanoma, head and neck cancer, liver cancer, intracranial tumors (such as gliomas), colorectal cancer, bladder cancer, breast cancer, ovarian cancer, uterine cancer, cervical cancer, gastric cancer, esophageal cancer, kidney cancer, prostate cancer, pancreatic cancer, lymphatic cancer, leukemia, bone cancer, testicular cancer and osteosarcoma.

In a preferred embodiment of the present invention, the administered dose of the recombinant oncolytic vaccinia virus is a therapeutically effective amount, once a day, for continuous administration for 1-6 days; or once every two days, for continuous administration for 1-6 times. The therapeutically effective amount is preferably a dose of 1 × 10⁵ to 5 × 10⁹ pfu/day (e.g., a dose of 1 × 10⁵ to 3 × 10⁹ pfu/day, a dose of 1 × 10⁵ to 1 × 10⁸ pfu/day, etc.).

If necessary, the recombinant oncolytic vaccinia virus of the present invention can also be used in combination with other drugs, such as interleukin-2 (IL-2), IL-15, IL-17, IL-18, granulocyte-macrophage colony-stimulating factor (GM-CSF), interferon-γ (IFN-γ), tumor necrosis factor-α (TNF-α), and the like, and the dosage and administration route thereof may be performed in their respective conventional manners.

Based on specific situations and needs, the method for treating tumors and/or cancers according to the present invention may be applied to a patient for one time or multiple times.

The oncolytic virus may be administered by the administration methods commonly used in the art, such as intratumoral injection or intravenous administration.

The invention also provides a therapeutic agent, comprising:
(A) a first pharmaceutical composition, wherein the first pharmaceutical composition comprises the recombinant oncolytic vaccinia virus of the present invention in a first pharmaceutically acceptable carrier; and
(B) a second pharmaceutical composition, wherein the second pharmaceutical composition comprises NK cells in a second pharmaceutically acceptable carrier.

In some embodiments, the first and second pharmaceutically acceptable carriers are the same. In other embodiments, the first pharmaceutically acceptable carrier and the second pharmaceutically acceptable carrier are different.

In some cases, the therapeutic agent can also be understood as a combination of drugs.

The mechanisms through which oncolytic viruses kill tumor cells are generally similar. In various embodiments, the oncolytic viruses are administered via intratumoral injection or administered intravenously, and when the oncolytic viruses come into contact with tumor cells, they will infect and enter the tumor cells. Since the oncolytic virus mainly replicates and reproduces in tumor cells with little to no replication in normal cells, large amounts of progeny oncolytic viruses can be produced in the infected tumor cells, leading to lysis and death of the tumor cells. When the tumor cells lyse, large numbers of tumor-associated antigens and the progeny oncolytic viruses may be released, and the antigens can then further activate the immune system *in vivo,* stimulating NK cells and T cells *in vivo* to continue to attack the remaining tumor cells. Meanwhile, the progeny oncolytic viruses can infect the tumor cells which have not been infected yet.

NK cells are immune cells which can kill a broad spectrum of tumor cells, and NK cells can distinguish tumor cells from normal cells. When NK cells come into contact with tumor cells, they can recognize tumor cells as abnormal cells, and will kill the tumor cells through multiple assisting processes such as receptor recognition, target recognition by antibodies (ADCC), as well as releases of granzymes, perforins and interferons capable of killing the tumor cells indirectly. An *in vitro* study has indicated that a healthy NK cell is able to kill up to 27 tumor cells during its life cycle.

NK cells also have anti-virus functions. If normal cells are infected by viruses, the viruses will replicate massively and the infected cells will become senescence, showing changes in the composition of protein clusters on the cellular membrane thereof. During this process, NK cells are able to recognize the infected cells sensitively and effectively, and kill these cells with similar approaches they use to kill tumor cells as described above, so as to inhibit replication and proliferation of viruses. Afterwards, with activation of antigens and participation of immune factors like interferons, other types of immune cells will continue to fight against viruses.

In the present invention, individual features of the oncolytic virus and NK cells have been taken into account, so they can be combined skillfully. When combined together, the anti-virus mechanism of NK cells is also applicable to tumor cells infected by the oncolytic virus, and this is complementary to the anti-tumor mechanism of NK cells. In addition, the combination therapy allows the tumor cells infected by the oncolytic viruses to become specific targets for NK cells, which can improve their tumor killing effect. The oncolytic viruses not only replicate selectively in cancer cells and kill them from inside, but may also cause the protein receptor clusters on the cellular membrane to change and thus facilitate the recognition of cancer cells by NK cells, so that NK cells can attack the cancer cells from outside. Therefore, the oncolytic virus and NK cells synergistically kill cancer cells, achieving improved efficacy. Further and preferably, the recombinant oncolytic vaccinia virus of the present invention also expresses exogenous IL-21, and the expressed exogenous IL-21 can enhance the killing power of NK cells, thereby further enhancing the killing effect of NK cells. The combined use of the recombinant oncolytic vaccinia virus and NK cells described in the present invention can generate surprising tumor killing effects.

Preferably, the active ingredient of the first pharmaceutical composition is the recombinant oncolytic vaccinia virus according to the present invention, and the active ingredient of the second pharmaceutical composition is the NK cells.

Preferably, the first pharmaceutical composition and the second pharmaceutical composition are each independently present in the therapeutic agent without being mixed with each other.

In the present invention, the NK cells may be selected from autologous NK cells and allogeneic NK cells; preferably, the NK cells are autologous NK cells obtained from *in vitro* expansion or allogeneic NK cells obtained from *in vitro* expansion. Large-scale *in vitro* expansion culture techniques of NK cells are known and have substantially matured (see, for example, the following scientific literature: "Somanchi SS, Lee DA. Ex Vivo Expansion of Human NK Cells Using K562 Engineered to Express Membrane Bound IL21 Methods Mol Biol. 2016; 1441: 175-93. " or " Phan MT, Lee SH, Kim SK, Cho D. Expansion of NK Cells Using Genetically Engineered K562 Feeder Cells. Methods Mol Biol. 2016; 1441: 167-74."). Clinical data confirmed that autologous NK cells, semi-allogeneic NK cells (belonging to allogeneic NK cells), and NK cells prepared from cord blood have no toxic side effects, no long-term dependence, and are safe and effective upon recirculating to the human body.

The purity range of NK cells that can be used for treatment may be: the purity of autologous NK cells may be 85% or more, the purity of allogeneic NK cells may be 90% or more; wherein the impurity cells may be NK-T and/or γδ T cells. Preferably, the NK cell activity (viability) is 90% or more, and the NK cell killing activity is 80% or more.

In the combined treatment scheme of the present invention, the present invention further explores and optimizes the respective administration doses of oncolytic virus and NK cells, which is essential. Preferably, the first pharmaceutical composition comprises the recombinant oncolytic vaccinia virus at a dose of 1 × 10⁵ to 5 × 10⁹ pfu/day (e.g., the recombinant oncolytic virus at a dose of 1 × 10⁵ to 3 × 10⁹ pfu/day, the recombinant oncolytic vaccinia virus at a dose of 1 × 10⁵ to 1 × 10⁸ pfu/day, etc.), and the second pharmaceutical composition contains the NK cells at a dose of 1 × 10⁷ to 1 × 10¹⁰ cells/day (preferably, the second pharmaceutical composition comprises the NK cells at a dose of 1 × 10⁸ to 5 × 10⁹ cells/day; further preferably, the second pharmaceutical composition comprises the NK cells at a dose of 1 × 10⁹ to 4 × 10⁹ cells/day; more preferably, the second pharmaceutical composition comprises the NK cells at a dose of 1 × 10⁹ to 3 × 10⁹ cells/day). Preferably, the active ingredient of the therapeutic agent is composed of the recombinant oncolytic vaccinia virus at a dose of 1 × 10⁵ to 5 × 10⁹ pfu/day (e.g., the recombinant oncolytic vaccinia virus at a dose of 1 × 10⁵ to 3 × 10⁹ pfu/day, the recombinant oncolytic vaccinia virus at a dose of 1 × 10⁵ to 1 × 10⁸ pfu/day, etc.) and the NK cells at a dose of 1 × 10⁷ to 1 × 10¹⁰ cells/day (e.g., 1 × 10⁸ to 5 × 10⁹ cells/day, 1 × 10⁹ to 4 × 10⁹ cells/day, 1 × 10⁹ to 3 × 10⁹ cells/day, etc.).

The recombinant oncolytic vaccinia virus may be administered by the administration methods commonly used in the art, such as intratumoral injection or intravenous administration.

The NK cells may be administered using a method of administration commonly used in the art, for example, intravenously.

Those skilled in the art will appreciate that the therapeutic agents of the present invention may further comprise suitable pharmaceutically acceptable excipients.

The therapeutic agent of the present invention may also contain other active ingredients known in the art, such as interleukin-2 (IL-2), IL-15, IL-17, IL-18, granulocyte-macrophage colony-stimulating factor (GM-CSF), interferon-γ (IFN-γ), tumor necrosis factor-α (TNF-α), etc.

In some embodiments, a therapeutic agent of the invention comprises one or more pharmaceutically acceptable carriers. The pharmaceutical formulations may be prepared by methods known in the art. For example, active ingredients such as compounds may be formulated with common excipients, diluents (such as phosphate buffer or saline), tissue culture media, and carriers (such as autologous plasma or human serum albumin) and be administrated as suspensions. Other carriers may include liposomes, micelles, nanocapsules, polymeric nanoparticles, solid lipid particles (see, for example, the reference "E. Koren and V. Torchilin, Life, 63: 586-595, 2011"). Specific methods of formulating the therapeutic agents of the present invention can be found in the scientific and patent literatures, for example, see the latest edition of Remington's Pharmaceutical Sciences, Maack Publishing Company, Easton PA ("Remington's").

The therapeutic agent of the present invention can be used for the treatment of a variety of tumors and/or cancers, including but not limited to: lung cancer, melanoma, head and neck cancer, liver cancer, intracranial tumors (such as gliomas), colorectal cancer, bladder cancer, breast cancer, ovarian cancer, uterine cancer, cervical cancer, gastric cancer, esophageal cancer, kidney cancer, prostate cancer, pancreatic cancer, lymphatic cancer, leukemia, bone cancer, testicular cancer and osteosarcoma.

The administration method of the therapeutic agent of the present invention is as follows: first, administering the recombinant oncolytic vaccinia virus to a tumor and /or cancer patient, 18-72 hours (e.g., 20-70 hours, 22-48 hours, 24-48 hours, 30-48 hours, etc.) after the administration of the recombinant oncolytic vaccinia virus, administering the NK cells to the tumor and/or cancer patient. The phrase "18-72 hours (e.g., 20-70 hours, 22-48 hours, 24-48 hours, 30-48 hours, etc.) after the administration of the recombinant oncolytic vaccinia virus, administering the NK cells to the tumor and/or cancer patient" means that the time interval between the first administration of the NK cells and the first administration of the recombinant oncolytic vaccinia virus is in the range of 18-72 hours (e.g., 20-70 hours, 22-48 hours, 24-48 hours, 30-48 hours, etc.), or the time interval between the first administration of the NK cells and the most recent administration of the recombinant oncolytic vaccinia virus is in the range of 18-72 hours (e.g., 20-70 hours, 22-48 hours, 24-48 hours, 30-48 hours, etc.). Preferably, the time interval between the first administration of the NK cells and the most recent administration of the recombinant oncolytic vaccinia virus is in the range of 18-72 hours (e.g., 20-70 hours, 22-48 hours, 24-48 hours, 30-48 hours, etc.). More preferably, the time interval between the first administration of the NK cells and the most recent administration of the recombinant oncolytic vaccinia virus is in the range of 24-48 hours.

In a preferred embodiment of the present invention, the recombinant oncolytic vaccinia virus is administered in a therapeutically effective amount, once a day for 1-6 consecutive days; and the NK cell is administered at a dose of 1 × 10⁷ to 1 × 10¹⁰ cells/day (e.g., a dose of 1 × 10⁸ to 5 × 10⁹ cells/day, a dose of 1 × 10⁹ to 4 × 10⁹ cells/day, a dose of 1 × 10⁹ to 3 × 10⁹ cells/day), once a day for 1-6 consecutive days. In another preferred embodiment of the present invention, the administration dose of the recombinant oncolytic vaccinia virus is a therapeutically effective amount, once every other day for continuous administration for 2-6 days; and the administration dose of the NK cells is 1 × 10⁷ to 1 × 10¹⁰ cells/day (e.g., a dose of 1 × 10⁸ to 5 × 10⁹ cells/day, a dose of 1 × 10⁹ to 4 × 10⁹ cells/day, a dose of 1 × 10⁹ to 3 × 10⁹ cells/day), once every other day, continuous administration for 2-6 days. Any one of the above mentioned embodiments or any other alternative embodiment can be adopted according to the present invention, as long as the NK cells are to be given to the tumor and/or cancer patient 18 to 72 hours after administration of the recombinant oncolytic vaccinia virus. The recombinant oncolytic vaccinia virus and NK cells may be administered alternatively (for example, administering the recombinant oncolytic vaccinia virus on Day 1, administering the NK cells on Day 2, administering the recombinant oncolytic vaccinia virus on Day 3, and administering the NK cells on Day 4, and so on); or may be administered sequentially (for example, administering the recombinant oncolytic vaccinia virus on Day 1, administering the recombinant oncolytic vaccinia virus and NK cells in a sequential order on Day 2, administering the recombinant oncolytic vaccinia virus and NK cells in a sequential order on Day 3, and administering the recombinant oncolytic vaccinia virus and NK cells in a sequential order on Day 4, and so on); or may be administered using other dosage regimens (for example, first, administering the recombinant oncolytic vaccinia virus once daily for consecutive 1 to 6 days, and after an interval of 18 to 72 hours, administering the NK cells once daily for consecutive 1 to 6 days). Preferably, the recombinant oncolytic vaccinia virus is administered first, and the NK cells are administered 18 to 72 hours after completion of administrating all the doses of recombinant oncolytic vaccinia virus. In a preferred embodiment of the present invention, first, the tumor and/or cancer patient is given the recombinant oncolytic vaccinia virus, and the recombinant oncolytic vaccinia virus is given once only at a therapeutically effective dose; 18 to 72 hours after administration of the recombinant oncolytic vaccinia virus, the tumor and/or cancer patient is administered the NK cells, and the NK cells are administered once only at a dose ranging from 1×10⁷ to 1×10¹⁰ cells/day (e.g., a dose of 1×10⁸ to 5×10⁹ cells/day, a dose of 1×10⁹ to 4×10⁹ cells/day, a dose of 1×10⁹ to 3×10⁹ cells/day). The therapeutically effective amount of the recombinant oncolytic vaccinia virus is preferably a dose of 1 × 10⁵ to 5 × 10⁹ pfu/day (e.g., a dose of 1 × 10⁵ to 3 × 10⁹ pfu/day, a dose of 1 × 10⁵ to 1 × 10⁸ pfu/day).

The invention also provides the use of the therapeutic agent of the invention in the preparation of drugs for treating tumors and/or cancers.

The tumors and/or cancers include, but are not limited to, lung cancer, melanoma, head and neck cancer, liver cancer, intracranial tumors (such as gliomas), colorectal cancer, bladder cancer, breast cancer, ovarian cancer, uterine cancer, cervical cancer, gastric cancer, esophageal cancer, kidney cancer, prostate cancer, pancreatic cancer, lymphatic cancer, leukemia, bone cancer, testicular cancer and osteosarcoma.

According to another aspect of the present invention, there is also provided a kit of combinational drugs with synergistic effect for treatment of tumors and/or cancers, including a first container containing the recombinant oncolytic vaccinia virus of the present invention and a second container containing the NK cells of the present invention, wherein the first container is separate from the second container. The kit further comprises an instruction specifying timing and routes of administration. Preferably, the kit consists of independent containers respectively containing the recombinant oncolytic vaccinia virus of the present invention and the NK cells of the present invention, and an instruction specifying timing and routes of administration.

The tumors and/or cancers include, but are not limited to, lung cancer, melanoma, head and neck cancer, liver cancer, intracranial tumors (such as gliomas), colorectal cancer, bladder cancer, breast cancer, ovarian cancer, uterine cancer, cervical cancer, gastric cancer, esophageal cancer, kidney cancer, prostate cancer, pancreatic cancer, lymphatic cancer, leukemia, bone cancer, testicular cancer and osteosarcoma.

Preferably, the first container containing the recombinant oncolytic vaccinia virus of the present invention contains a therapeutically effective amount of the recombinant oncolytic vaccinia virus, and the second container containing the NK cells contains the NK cells sufficient to provide a dose of 1 × 10⁷ to 1 × 10¹⁰ cells/day (for example, the NK cells at a dose of 1 × 10⁸ to 5 × 10⁹ cells/day, the NK cells at a dose of 1 × 10⁹ to 4 × 10⁹ cells/day, the NK cells at a dose of 1 × 10⁹ to 3 × 10⁹ cells/day, etc.). The therapeutically effective amount of the recombinant oncolytic vaccinia virus is preferably a dose of 1 × 10⁵ to 5 × 10⁹ pfu/day (e.g., a dose of 1 × 10⁵ to 3 × 10⁹ pfu/day, a dose of 1 × 10⁵ to 1 × 10⁸ pfu/day).

Preferably, the first container containing the recombinant oncolytic vaccinia virus contains the recombinant oncolytic vaccinia virus at a dose of 1 × 10⁵ to 1 × 10⁸ pfu/day, and the second container containing the NK cells contains the NK cells at a dose of 1 × 10⁹ to 3 × 10⁹ cells/day.

In the present invention, the NK cells may be selected from autologous NK cells and allogeneic NK cells; preferably, the NK cells are autologous NK cells obtained from *in vitro* expansion or allogeneic NK cells obtained from *in vitro* expansion.

The recombinant oncolytic vaccinia viruses may be administered using their respective administration methods commonly used in the art, such as intratumoral injection or intravenous administration.

The NK cells may be administered using the method of administration commonly used in the art, for example, intravenously.

The tumors and/or cancers include, but are not limited to, lung cancer, melanoma, head and neck cancer, liver cancer, intracranial tumors (such as gliomas), colorectal cancer, bladder cancer, breast cancer, ovarian cancer, uterine cancer, cervical cancer, gastric cancer, esophageal cancer, kidney cancer, prostate cancer, pancreatic cancer, lymphatic cancer, leukemia, bone cancer, testicular cancer and osteosarcoma.

Another aspect of the present invention also provides a method for treating tumors and/or cancers, comprising, in a sequential manner, the following steps:
1) administering the recombinant oncolytic vaccinia viruses according to the present invention to a tumor and/or cancer patient;
2) 18-72 hours (e.g., 20-70 hours, 22-48 hours, 24-48 hours, 30-48 hours, etc.) after the administration of the recombinant oncolytic vaccinia viruses, administering the NK cells according to the present invention to the tumor and/or cancer patient.

The phrase "18-72 hours (e.g., 20-70 hours, 22-48 hours, 24-48 hours, 30-48 hours, etc.) after the administration of the recombinant oncolytic vaccinia viruses, administering the NK cells according to the present invention to the tumor and/or cancer patient" means that the time interval between the first administration of the NK cells and the first administration of the recombinant oncolytic vaccinia viruses is in the range of 18-72 hours (e.g., 20-70 hours, 22-48 hours, 24-48 hours, 30-48 hours, etc.), or the time interval between the first administration of the NK cells and the most recent administration of the recombinant oncolytic vaccinia viruses is in the range of 18-72 hours (e.g., 20-70 hours, 22-48 hours, 24-48 hours, 30-48 hours, etc.). Preferably, the time interval between the first administration of the NK cells and the most recent administration of the recombinant oncolytic vaccinia viruses is in the range of 18-72 hours (e.g., 20-70 hours, 22-48 hours, 24-48 hours, 30-48 hours, etc.). More preferably, the time interval between the first administration of the NK cells and the most recent administration of the recombinant oncolytic vaccinia viruses is in the range of 24-48 hours.

The tumors and/or cancers include, but are not limited to, lung cancer, melanoma, head and neck cancer, liver cancer, intracranial tumors (such as gliomas), colorectal cancer, bladder cancer, breast cancer, ovarian cancer, uterine cancer, cervical cancer, gastric cancer, esophageal cancer, kidney cancer, prostate cancer, pancreatic cancer, lymphatic cancer, leukemia, bone cancer, testicular cancer and osteosarcoma.

The oncolytic viruses can selectively replicate in tumors or cancer cells and reach a peak after a certain period of time. The inventors of the present invention have found that after a period of replication, the oncolytic virus in tumor cells can promote the killing of tumor cells by NK cells. Therefore, the administration sequence and interval of the recombinant oncolytic vaccinia virus and NK cells proposed by the present invention achieve a double-peak overlap of the peaks of the two effects.

The present invention further explores and optimizes the respective administration doses of the recombinant oncolytic vaccinia virus and NK cells, and the coordination thereof with the above-mentioned administration sequence and interval is crucial, which determines the anti-tumor efficacy of the recombinant oncolytic vaccinia virus, anti-tumor efficacy of NK cells, and the best synergistic killing against tumor cells of the above two.

In a preferred embodiment of the present invention, the recombinant oncolytic vaccinia virus is given at a therapeutically effective dose once daily, consecutively for 1 to 6 days; and the NK cells are given at a dose ranging from 1×10⁷ to 1×10¹⁰ cells/day (e.g., a dose of 1×10⁸ to 5×10⁹ cells/day, a dose of 1×10⁹ to 4×10⁹ cells/day, a dose of 1×10⁹ to 3×10⁹ cells/day) once daily, consecutively for 1 to 6 days. In another preferred embodiment of the present invention, the recombinant oncolytic vaccinia virus is given at a therapeutically effective dose, once every other day, consecutively for 2 to 6 days; and the NK cells are given at a dose ranging from 1×10⁷ to 1×10¹⁰ cells/day (e.g., a dose of 1×10⁸ to 5×10⁹ cells/day, a dose of 1×10⁹ to 4×10⁹ cells/day, a dose of 1×10⁹ to 3×10⁹ cells/day), once every other day, consecutively for 2 to 6 days. Any one of the above mentioned embodiments or any other alternative embodiment can be adopted according to the present invention, as long as the NK cells are to be given to the tumor and/or cancer patient 18 to 72 hours after administration of the recombinant oncolytic vaccinia virus. The recombinant oncolytic vaccinia virus and NK cells may be administered alternatively (for example, administering the recombinant oncolytic vaccinia virus on Day 1, administering the NK cells on Day 2, administering the recombinant oncolytic vaccinia virus on Day 3, and administering the NK cells on Day 4, and so on); or may be administered sequentially (for example, administering the recombinant oncolytic vaccinia virus on Day 1, administering the recombinant oncolytic vaccinia virus and NK cells in a sequential order on Day 2, administering the recombinant oncolytic vaccinia virus and NK cells in a sequential order on Day 3, and administering the recombinant oncolytic vaccinia virus and NK cells in a sequential order on Day 4, and so on); or may be administered using other dosage regimens (for example, first, administering the recombinant oncolytic vaccinia virus once daily for consecutive 1 to 6 days, and after an interval of 18 to 72 hours, administering the NK cells once daily for consecutive 1 to 6 days). Preferably, the recombinant oncolytic vaccinia virus is administered first, and the NK cells are administered 18 to 72 hours after completion of administrating all the doses of recombinant oncolytic vaccinia virus. In a preferred embodiment of the present invention, first, the tumor and/or cancer patient is given the recombinant oncolytic vaccinia virus, and the recombinant oncolytic vaccinia virus is given once only at a therapeutically effective dose; 18 to 72 hours after administration of the recombinant oncolytic vaccinia virus, the tumor and/or cancer patient is given the NK cells, and the NK cells are given once only at a dose ranging from 1×10⁷ to 1×10¹⁰ cells/day (e.g., a dose of 1×10⁸ to 5×10⁹ cells/day, a dose of 1×10⁹ to 4×10⁹ cells/day, a dose of 1×10⁹ to 3×10⁹ cells/day). The therapeutically effective amount of the recombinant oncolytic vaccinia virus is preferably 1 × 10⁵ to 5 × 10⁹ pfu/day (e.g., a dose of 1 × 10⁵ to 3 × 10⁹ pfu/day, a dose of 1 × 10⁵ to 1 × 10⁸ pfu/day and so on).

Based on specific situations and needs, the method for treating tumors and/or cancers according to the present invention may be applied to a patient for one time or multiple times.

In the present invention, the NK cells may be selected from autologous NK cells and allogeneic NK cells; preferably, the NK cells are autologous NK cells obtained from *in vitro* expansion or allogeneic NK cells obtained from *in vitro* expansion.

The tumors and/or cancers include lung cancer, melanoma, head and neck cancer, liver cancer, intracranial tumors (such as gliomas), colorectal cancer, bladder cancer, breast cancer, ovarian cancer, uterine cancer, cervical cancer, gastric cancer, esophageal cancer, kidney cancer, prostate cancer, pancreatic cancer, lymphatic cancer, leukemia, bone cancer, testicular cancer and osteosarcoma.

The recombinant oncolytic vaccinia virus may be administered by the administration methods commonly used in the art, such as intratumoral injection or intravenous administration.

The NK cells may be administered using a method of administration commonly used in the art, for example, intravenously.

Hereinafter, the present invention will be further explained or described by way of examples, but these examples are not intended to limit the scope of protection of the present invention.

### Examples

Unless otherwise specified, the experimental methods used in the following examples are performed using conventional experimental procedures, operations, materials, and conditions in the field of biological engineering.

Unless otherwise specified, all the percentage concentrations (%) of the respective agents indicate percentage by volume [%(v/v)].

The biological materials used in the following examples are as follows:
1. CV1 African green monkey kidney cells (culture medium: 10% FBS + MEM + 1% P/S) are purchased from ATCC. MRC-5 normal human embryonic lung fibroblasts (culture medium: 10% FBS + DMEM + 1% P/S) are purchased from the Chinese Academy of Sciences Shanghai Cell Bank. HEK-293 and HEK-293T human embryonic kidney cells (culture medium: 10%FBS + DMEM + 1% P/S) are purchased from Agilent Technologies and the Chinese Academy of Sciences Shanghai Cell Bank, respectively. HUVEC human normal umbilical vein endothelial cells (culture medium: 10%FBS + H-004B + 1%P/S) were purchased from Allcells Biotechnology (Shanghai) Co., Ltd.
2. Tumor cells: The source of tumor cellines and cell culture medium are shown in Table A below

**Table A**

| Cell Line | Source | Medium |
|---|---|---|
| HCT116 | ATCC | 10%FBS +McCoy's 5A+1%P/S |
| SKOV3 | Chinese Academy of Sciences Shanghai Cell Bank | 10%FBS +McCoy's 5A+1%P/S |
| SaoS2 | Chinese Academy of Sciences Shanghai Cell Bank | 10%FBS +McCoy's 5A+1%P/S |
| SK-BR-3 | ATCC | 10%FBS +McCoy's 5A+1%P/S |
| U251 | Chinese Academy of Sciences Shanghai Cell Bank | 10%FBS +DMEM+1%P/S |
| PANC-1 | ATCC | 10%FBS +DMEM+1%P/S |
| SK-HEP-1 | Chinese Academy of Sciences Shanghai Cell Bank | 10%FBS +MEM+1%P/S |
| FaDu | Hangzhou First People's Hospital (China) | 10%FBS +MEM+1%P/S |
| MNNG/HOS C1 | ATCC | 10%FBS +MEM+1%P/S |
| Hela | ATCC | 10%FBS +MEM+1%P/S |
| HepG2 | Chinese Academy of Sciences Shanghai Cell Bank | 10%FBS +MEM+1%P/S |
| MCF-7 | ATCC | 10%FBS +MEM+1%P/S |
| C33A | Wuhan Cell Bank | 10%FBS +MEM+1%P/S |
| A549 | ATCC | 10%FBS +F12K+1%P/S |
| LOVO | ATCC | 10%FBS +F12K+1%P/S |
| CFPAC-1 | ATCC | 10%FBS +IMDM+1%P/S |
| CV1 | ATCC | 10%FBS +MEM+1%P/S |
| NCI-H1299 | ATCC | 10%FBS +RPMI1640 |
| U87MG | ATCC | 10%FBS +MEM |

McCoy's 5A, F12K, IMDM, DMEM, RPMI1640 and MEM were purchased from GIBCO. Fetal bovine serum FBS was purchased from SIGMA, and penicillin-streptomycin (P/S) solution (100X) was purchased from Beyotime Biotechnology.
3. Virus: Backbone vector VSC20 vaccinia virus is a vaccinia virus with VGF gene deletion, the preparation method can be found in the scientific literature: "McCart, JA, et al. Systemic cancer therapy with a tumor-selective vaccinia virus mutant lacking thymidine kinase and vaccinia growth factor genes. Cancer Res (2001) 61: 8751-8757". DDvv-hIL21 virus was genetically modified, including the use of artificial synthetic vaccinia virus early/late promoter pSEL to regulate the expression of exogenous human IL21 gene, and the use of intracellular recombination technology to insert the human IL21 gene into the TK gene region of the vaccinia virus VSC20 strain, thereby constructing a double-deleted oncolytic vaccinia virus DDvv-hIL21 (see Chinese Patent Application Publication No. CN109554353A).
The oncolytic vaccinia virus DDVV-RFP as the control virus is belongs to the oncolytic vaccinia virus WR strain (see, for example, "X Song, et al. T-cell Engager-armed Oncolytic Vaccinia Virus Significantly Enhances Antitumor Therapy. Molecular Therapy. (2014); 22 1, 102-111"). The virus is functionally deficient in both TK gene and VGF gene, and carries an exogenous red fluorescent protein (RFP) gene. Since the RFP gene only plays a role for screening/reporting, the anti-tumor function of the oncolytic vaccinia virus DDvv-RFP is substantially equivalent to the oncolytic vaccinia virus functionally deficient in TK gene and VGF gene. Also, the oncolytic vaccinia virus DDvv-RFP is obtained by genetic modification of VSC20 vaccinia virus using conventional techniques in the art. VSC20 vaccinia virus is a vaccinia virus lack of VGF gene. For the preparation method of VSC20 vaccinia virus, see "McCart, JA, et al. Systemic cancer therapy with a tumor-selective vaccinia virus mutant lacking thymidine kinase and vaccinia growth factor genes. Cancer Res (2001) 61: 8751-8757". The genetic modification involves the use of an artificial synthetic vaccinia virus early/late promoter pSEL to regulate the expression of exogenous RFP gene, and insertion of the RFP gene into the TK gene region of the vaccinia virus VSC20 strain using an *in vitro* intracellular recombination technique, thereby constructing the oncolytic vaccinia virus DDVV-RFP.
4. C57BU6 mice and BalBc-Nude mice were purchased from Beijing Vital River Laboratory Animal Technology Co., Ltd. Severe immunodeficiency NCG mice were purchased from Jiangsu GemPharmatech Co., Ltd.
5. Culture plate: 6-well cell culture plates, 24-well cell culture plates, and 96-well cell culture plates were purchased from Corning Co., Ltd.
6. Preparation of gpt drugs: 10 mg/mL of mycophenolic acid (400 ×), 10 mg/mL of 40 × xanthine (40 ×) and 10 mg/mL of hypoxanthine (670 ×) were prepared using 0.1N NaOH, respectively, and stored in dark at -20°C. An 1× working solution was prepared by adding 100 µL of 400× mycophenolic acid, 40 µL of 40× xanthine and 60 µL of 670× hypoxanthine into 40 mL of DMEM, mixing well, filtering with a 0.22 µm filter and storing at 4°C before using.
7. PBS formula: 8mM Na₂HPO₄, 136mM NaCl, 2mM KH₂PO₄, 2.6mM KCI, pH 7.2-7.4.
8. Virus purification solution formula: 60% (w/v), 50% (w/v), 40% (w/v), 30% (w/v) sucrose solution.

The cell counting method used in the following examples is as follows:
MTT assay: 10µL of MTT solution (5 mg/mL) was added to cells in each well, then the cells were incubated in an incubator at 37°C for 4 to 6 hours, the culture medium was discarded, 150 µL of DMSO was added into each well, and then shaken at low speed on a shaker for 10 minutes, allowing the crystal substance to be fully dissolved, and the absorbance value at 490 nm (OD₄₉₀) was measured using a microplate reader. Calculation formula of inhibition rate: Cell proliferation Inhibitory Rate (IR%) = 1- (OD₄₉₀ tested product-OD₄₉₀ blank) / (OD₄₉₀ negative control-OD₄₉₀ blank) × 100%.

The abbreviations used in the following examples are explained below:
miR199T: target sequence of miR199 (hsa-miR-199a-3p or hsa-miR-199b-3p)
FBS: Fetal Bovine Serum
P/S: Penicillin-Streptomycin
mCherry/Zeocin: Red fluorescence protein /Bleomycin
DMSO: Dimethyl sulfoxide

### Preparation Example 1: Construction of pZB-E10R-miR199T plasmid

In order to construct the shuttle plasmid pZB-E10R-miR199T carrying 4 repeated miR199T sequences. Firstly, 4 × miR199T repeat fragments were synthesized by gene synthesis method, and each fragment was linked with GG, and inserted into the 3'UTR region of E10R locus of vaccinia virus (the insertion site is at the 56974 bp of the left homologous arm of the E10R gene). Then, the mCherry/Zeocin gene driven by mH5 promoter was inserted, both 5' end and 3' end of which respectively comprising a LoxP sequence in the same direction. The DNA sequence (SEQ ID NO.1) of miR199T is as follows: 5'-acagtagtctgcacattggtta-3' (the miRBase database accession number of the corresponding miRNA mature form is MIMAT0000232 (hsa-miR-199a-3p) or MIMAT0004563 (hsa-miR-199b-3p)).

In summary, pZB-E10R-miR199T was obtained by enzyme cleavage and homologous recombination by using pcDNA3.1(+)-E10R-mCherry/Zeocin containing the left and right homologous arms of gene E10R of the vaccinia virus and mCherry/Zeocin gene, plasmid pUC-57-miR199T containing four repeats of miR199T (constructed and synthesized by Beijing Tsingke Biotechnology Co., Ltd.), and plasmid pCB (it was obtained according to the reference "Yourong FANG et al, Construction of Recombinant Vaccinia Virus Vector with Zeocin and GFP Double Screening Labels, Journal of international epidemiology and infectious diseases, 2012. 39 (3): 148-152. ") (as shown in Figure 1).

The specific operation is as follows:
The following PCR procedure can be carried out with appropriate adjustment according to the actual situation under the temperature and cycle conditions within the conventional range, which belongs to the conventional technology in the art.
1) Construction of pcDNA3.1(+)-E10R-mCherry/Zeocin plasmid: The vaccinia virus Dvv-VSC20 genome (the source of the virus is described in the reference "McCart, JA, et al. Systemic cancer therapy with a tumor-selective vaccinia virus mutant lacking thymidine kinase and vaccinia growth factor genes. Cancer Res (2001) 61: 8751-8757.") was used as a template for PCR amplification [primer sequences are as follows: L-armE10: AGTCCTCGAGCTAATATTGAGAAATTCATC (SEQ ID NO. 2); E10R1: CTGCACATTGGTTATTAAGAAGCATAGTCTGGAACATCATATGGATATAAAGGGTTA ACCTTTGTC (SEQ ID NO. 3)] to obtain the E10R gene and the left homologous arm of fragment F1. Fragment F1 was used as a template for PCR amplification (primer sequences are as follows: L-armE10: AGTCCTCGAGCTAATATTGAGAAATTCATC [SEQ ID NO. 2); E10R2: TGGTTAGGACAGTAGTCTGCACATTGGTTAGGACAGTAGTCTGCACATTGGTTAT TAAG (SEQ ID NO. 4) ]to obtain the left homologous arm fragment F2 of E10R. The pCBmCZ-tTFNGR plasmid (it was obtained according to reference "Yourong FANG et al, Construction of Recombinant Vaccinia Virus Vector with Zeocin and GFP Double Screening Labels, Journal of international epidemiology and infectious diseases, 2012. 39 (3): 148-152.") was used as a template for PCR amplification [(primer sequences are as follows: 199H5: ATGTGCAGACTACTGTCCTAACCAATGTGCAGACTACTGTCCAAAAATTGAAAATA AATAC (SEQ ID NO.5); Zeo-rev, GATCAAGATCTTTAGTCCTGCTCCTCGGCCAC (SEQ ID NO.6) ]to obtain fragment F3 containing mH5 promoter and mCherry/Zeocin. The vaccinia virus Dvv-VSC20 genome was used as a template for PCR amplification (primer sequences are as follows: E11 UP: GATCAAGATCTTTATAAACTTAACCCATTATAAAAC (SEQ ID NO. 7); R-arm, AGTCGGATCCTTGACAGTCTTGAACAATTATAC (SEQ ID NO. 8)) to obtain F4 containing right homologous arm of E10R of the vaccinia virus. The fragments F2 and F3 were phosphorylated and ligated by T4 DNA ligase (Thermo, E10011). The ligated fragment was used as a template for PCR amplification (primer sequences are as follows: L-armE10, AGTCCTCGAGCTAATATTGAGAAATTCATC (SEQ ID NO. 2); Zeo-rev, GATCAAGATCTTTAGTCCTGCTCCTCGGCCAC (SEQ ID NO. 6)) to obtain a fusion fragment F5 containing the left homologous arm of E10R and mCherry/Zeocin sequence. Fragments F4 and F5 were digested with restriction endonuclease Bglll (Thermo, ER0082), respectively, and then ligated by T4 DNA ligase to obtain the E10R-mCherry/Zeocin fragment containing the left and right homologous arms of E10R and the mCherry/Zeocin sequence. The E10R-mCherry/Zeocin fragment and plasmid pCDNA3.1(+) (Invitrogen) were digested with restriction endonucleases BamHl (Thermo, FD0054) and Xhol (Thermo, FD0694), respectively, and then ligated by T4 DNA ligase, and transformed into *E. coli* DH5a (TIANGEN, CB101). A single colony was picked to obtain the pcDNA3.1(+)-E10R-mCherry/Zeocin plasmid, which was confirmed by sequencing.
2) Construction of pZB-1 plasmid: the pCB plasmid was used as a template for PCR amplification (primer sequences used are: zP12-F: CCGCTCGAGGCTGGCGTTTTTCCATAGG (SEQ ID NO. 33); zP12-R: CGCggatccCGGTCTGGTTATAGGTACATTGAG (SEQ ID NO. 34))to obtain a linearized L-pCB fragment. pcDNA3.1(+)-E10R-mCherry/Zeocin and L-pCB were double digested with restriction endonucleases BamHI and Xhol to obtain L-E10RE11 LID and L-pCB/D fragments, then ligated by T4 DNA ligase, and finally transformed into *E. coli* DH5a. A single colony was picked to obtain the pZB-1 plasmid, which was confirmed by sequencing.
3) Construction of pZB-2 plasmid: the pZB-1 plasmid was used as a template for PCR amplification [primer sequences are as follows: zP13-F: CCCAAGCTTTTAGTCCTGCTCCTCGGCC (SEQ ID NO. 9); zP11m-R: ATAACTTCGTATAGCATACATTATACGAAGTTATCTTTATAAACTTAACCCATTATAAA AC (SEQ ID NO. 10)) to obtain L-pZB-1 fragment (4815bp), in which LoxP sequence [ATAACTTCGTATAATGTATGCTATACGAAGTTAT (SEQ ID NO. 11)] was inserted into the 3' end of mCherry/Zeocin. The resulting L-pZB-1 fragment was self-ligated by T4 DNA ligase, transformed into *E. coli* DH5α, and a single colony was picked to obtain the pZB-2 plasmid, which was confirmed by sequencing.
4) Construction of pZB-E10R-miR199T plasmid: the pZB-2 plasmid was used as a template for PCR amplification [primer sequences are as follows: zP11-F: CGCGTATTTGGGATCAGATG (SEQ ID NO. 12); zP11-R: CAAAGGTTCTTGAGGGTTGTG (SEQ ID NO. 13)) to obtain a fragment (4689 bp) containing L-E10R-E11L-LoxP. pUC-57-miR199T was double digested with restriction endonucleases Kpnl (Thermo, FD0524) and Sacl (Thermo, FD1133) to obtain a L-miR199T fragment : CAATTTAACACAACCCTCAAGAACCTTTGTATTTATTTTCAATTTTTATAACTTCGTA TAATGTATGCTATACGAAGTTATGGACAGTAGTCTGCACATTGGTTAGGACAGTAG TCTGCACATTGGTTAGGACAGTAGTCTGCACATTGGTTAGGACAGTAGTCTGCAC ATTGGTTATTAAGAAGCATAGTCTGGAACATCATATGGATATAAAGGGTTAACCTTT GTCACATCGATCGCGTATTTGGGATCAGATGGTAC (SEQ ID NO.35). By using a one-step cloning method (TreliefΓM sosoo cloning kit ver.2, Beijing Tsingke Biotechnology Co., Ltd., TSV-S2), it was ligated with the fragment (4689bp) containing L-E10R-E11L-LoxP, transformed into *E. coli* DH5α, and a single colony was picked to obtain the pZB-E10R-miR199T plasmid. The verification of plasmid was performed using BamH I and Sma I (Thermo, FD0663) digestion (Figure 2). Primers Z10, Z11, Z14 and Z15 (Beijing Tsingke Biotechnology Co., Ltd.) were used to confirm the correctness by sequencing (sequences of primers are shown in Table 1).

**Table 1**

| Primer | Sequence 5'-3' | Company |
|---|---|---|
| Z10 | GTTGATCGGCACGTAAGAGG (SEQ ID NO.14) | Beijing Tsingke Biotechnology Co., Ltd. |
| Z11 | GTCACAGAAACCATGCCGTGTAG (SEQ ID NO.15) | |
| Z14 | GGAGCTTCCAGGGGGAAAC (SEQ ID NO.16) | |
| Z15 | GACCTCAGCGTCGTAGTGG (SEQ ID NO.17) | |
| zP19 | CCAATTAATGCACCGAACATC (SEQ ID NO.18) | |
| zP20 | ATGAATTGGAGGAGATGGTGG (SEQ ID NO.19) | |
| T7 | TAATACGACTCACTATAGGG (SEQ ID NO.20) | |
| P1 | ATCGCATTTTCTAACGTGATGGAT (SEQ ID NO.21) | |
| P2 | TATCTAACGACACAACATCCATT (SEQ ID NO.22) | |
| P5 | GCACGGTAAGGAAGTAGATC (SEQ ID NO.23) | |
| P18 | CAGTACACGGTCCTCAGCATAA (SEQ ID NO.24) | |

### Preparation Example 2: Packaging and identification of VSC20-mT/mCherry virus

1) Recombination of oncolytic vaccinia virus: a) CV1 cells were placed in a 6-well plate at 4×10⁵ cells/well, such that the cell density reached 80%-90% the next day. The MEM in the 6-well plate was discarded. 1 mL of serum-free and antibiotic-free MEM medium containing 8 × 10³ pfu of the Dvv-VSC20 virus was added to each well, such that the infection concentration reached 0.02MOI. After an incubation of 2 hours at 37°C, the culture medium was removed and replaced with 2% FBS+1% P/S MEM medium, and 300 µL of plasmid/Lipo3000 (LipofectamineOR 3000 transfection reagent, Invitrogen^{™}, L3000-015) mixed solution (Solution A: 2 µg of pZB-E10R-miR199T plasmid and 4 µL of P3000 reagent (Invitrogen^{™}, L3000-015) were added to 300 µL of Opti-MEM I and mixed well, and incubated for 5 minutes at room temperature; Solution B: 10 µL of Lipo3000, added to 300 µL of Opti-MEM I and mixed well, and incubated for 5 minutes at room temperature; solution A and solution B were mixed, and incubated for 15 minutes at room temperature) was added, and placed in an incubator (37°C, 5% CO₂) to continue culturing. b) when the cells were completely cytopathic observed under microscope after 24-48 hours, the supernatant and cells were collected, cell lysis by repeating frozen/thawed procedure for 3 times, virus was collected by centrifugation at 300g for 5 minutes and labelled as P0 generation. c) 200 µL of virus was applied for viral genome extraction using TIANamp Virus DNA/RNA kit (TIANGEN, DP315). It was confirmed by PCR with primers zP19/zP20 that the recombination of VSC20-mT/mCherry get success.
2) Screening of monoclonal recombinant oncolytic vaccinia viruses: a) CV1 cells were placed in five 100 mm petri dishes, such that the cell density reached 80%-90% the next day. The MEM medium was discarded, and 3 mL of serially diluted P0 virus in serum-free culture medium (with a gradient dilution of 10⁻¹-10⁻⁵) was added to each petri dish respectively. The petri dishes were placed in an incubator for 2 hours at 37°C, 5% CO₂, and cross-mixed every 30 minutes. After another incubation for 2 hours, the medium was discard, and 10 mLof 2% (w/v) agarose gel (MEM medium containing 2% FBS) was added. After solidification, it was placed in an incubator to continue culturing at 37°C, 5% CO₂. b) After about 48 hours, a single plaque with red fluorescence was picked into 200 µL of sterile PBS, frozen and thawed repeatedly for 3 times to release the virus. 100 µL of virus solution was taken out from each plaque and put in a 24-well plate. CV1 cells (cell density reached 80%) were used for expansion. After about 48 hours, when the cells were completely cytopathic, the viruses were collected and labelled as P1-X (X is the number 1, 2, 3, 4..., representing different virus clones). 200 µL of virus solution from each plaque was applied for viral genome extraction by using TIANamp Virus DNA/RNA kit, and then PCR was performed for identification. The rest of the virus solution was stored at -80°C. The above screening steps were repeated four times to obtain the P5 generation virus. Primers zP19 and zP20 (sequences of primers are shown in Table 1) were used for PCR to obtain a band of 2310bp, suggesting the confirmation of the VSC20-mT/mCherry monoclonal recombinant virus (Figure 3). The correctness of sequence was confirmed by Z11, Z15, zP19 and zP20 sequencing (sequences of primers are shown in Table 1). The VSC20-mT/mCherry viral vector is VGF gene functionally deficient and inserted with 4 repeated miR199T sequences and the mCherry red marker gene for screening in the 3'UTR region of the E10R gene (Figure 4).

### Preparation Example 3: Packaging and identification of MiTDvv-mCherry-backbone viruses

1) Recombination of oncolytic vaccinia virus: CV1 cells were placed in a 6-well plate at 4×10⁵ cells/well, such that the cell density reached 80%-90% confluence the next day. The MEM medium in the 6-well plate was discarded, and 1 mL of serum-free and antibiotic-free MEM medium containing 8 × 10³ pfu of the VSC20-mT/mCherry virus was added to each well, such that the infection concentration reached 0.02MOI. After an incubation of 2 hours at 37°C, the culture medium was discarded and replaced with 2%FBS+1% P/S MEM medium, and 300µL of pCB plasmid/Lipo3000 mixed solution (prepared as in Preparation Example 2) was added. When the cells were completely cytopathic observed by the microscope after 24-48 hours, the supernatant and cells were collected, frozen and thawed repeatedly for 3 times, centrifuged at 300g for 5 minutes, and the virus released in the supernatant was collected and labelled as P0 generation virus. The virus genome was extracted by using TIANamp Virus DNA/RNA kit (TIANGEN, DP315). It was confirmed by PCR with primers P5/P18 that the recombination of MiTDvv-mCherry virus get success.
2) gpt drug screening: A 60 mm petri dish was used to culture CV1 cells, and when it reached 80%, 1 mL of virus diluent diluted with MEM (containing 150 µL of P0 generation virus) was added. After 2 hours of infection, 4 mL of screening reagent containing 0.5 × gpt were added for screening. The cytopathy were observed, and the viruses were collected and labelled as the P1 generation. The above steps were repeated twice to obtain the P3 generation virus.
3) Screening of monoclonal recombinant oncolytic vaccinia viruses: The specific operation is the same as that in Preparation Example 2. After 4 hours of the infection of CV1 cells by serially diluted P3 virus, 10 mL of 2% (w/v) agarose gel was added to fix and culture the virus for about 48 hours, and a single plaque with red fluorescence was selected for PCR identification. According to the PCR results, positive clones were selected for repeated screening of P4-P5 generation, and MiTDvv-mCherry monoclonal virus was obtained. PCR identification confirmed that a band of 1840bp (sequences of primers P5 and P8 are shown in Table 1) was obtained, while a band of 622bp (sequences of primers P1 and P2 are shown in Table 1) was not obtained (Figure 5). Primers P5 and P18 were used for sequencing detection (Beijing Tsingke Biotechnology Co., Ltd.) to confirm that MiTDvv-mCherry backbone virus is both TK gene functionally deficient and VGF gene functionally deficient, with 4 repeats of miR199T sequence and mCherry red marker gene inserted into the 3'UTR region of the E10R gene, and carrying the gpt screening gene in the TK gene region (Figure 6).

### Preparation Example 4: Packaging and Identification of MiTDvv-hIL21-mCherry Oncolytic Virus

1) Recombination of oncolytic vaccinia virus: CV1 cells were spread in a 6-well plate at 4×10⁵ cells/well, such that the cell density reached 80%-90% the next day. The MEM medium in the 6-well plate was discarded, and 1 mL of serum-free and antibiotic-free MEM medium containing 8×10³ pfu of DDvv-hIL21 virus (the preparation method is as described in Chinese Patent Publication No.CN109554353A, the entire content of which is incorporated herein by reference) was added to each well such that the infection concentration reached 0.02 MOI. Cells were incubated for 2 hours at 37°C, and the culture medium was discarded, replaced with 2% FBS+1% P/S MEM medium. 300 µL of pZB-E10R-miR199T plasmid/Lipo3000 mixed solution (prepared as in Preparation Example 2) was added. When the cells were completely cytopathic observed by the microscope after 24-48 hours, the supernatant and cells were collected, frozen and thawed repeatedly for 3 times, centrifuged at 300g for 5 minutes, and the virus released in the supernatant was collected and labelled as P0 generation. The viral genome was extracted using TIANamp Virus DNA/RNA kit (TIANGEN, DP315). It was confirmed by PCR with primers zP19/zP20 that the recombination of MiTDvv-hIL21-mCherry oncolytic virus get success.
2) Screening of monoclonal recombinant oncolytic vaccinia viruses: the specific operation is the same as that described in Preparation Example 2). After 4 hours of the infection of CV1 cells with the serially diluted P0 generation virus, 10 mL of 2% (w/v) agarose gel was added to fix and culture the virus for about 48 hours, and a single plaque with red fluorescence was selected for PCR identification. According to the PCR results, positive clones were selected for repeated screening of P2-P5 generation, and monoclonal viruses were obtained. PCR identification confirmed that a band of 2310 bp was obtained (sequences of primers zP19 and zP20 are shown in Table 1). The correctness of sequence was confirmed by sequencing using Z11, Z15, zP19 and zP20 (sequences of primers are shown in Table 1) (Beijing Tsingke Biotechnology Co., Ltd.). MiTDvv-hIL21-mCherry oncolytic virus is both VGF gene functionally deficient and TK gene functionally deficient, with 4 repeats of miR199T and mCherry red marker gene inserted into the 3'UTR region of E10R gene, and carrying the IL-21 functional gene in the TK gene region (Figure 8).

### Preparation Example 5: Preparation of MiTDvv backbone virus and MiTDvv-hIL21 oncolytic virus by Cre-LoxP cleavage system

Obtaining of MiTDvv backbone virus (MiTDvv-mCherry virus with mCherry/Zeocin sequence deleted) and MiTDvv-hIL21 oncolytic virus (MiTDvv-hIL21-mCherry virus with mCherry/Zeocin sequence deleted):
1) CV1 cells were placed in a 6-well plate with 4×10⁵ cells/well, such that the cell density reached about 70% confluence at next day. The medium was replaced with 5%FBS+1%P/S MEM medium, and 300 µL of pBS185 CMV-Cre plasmid (Cre gene is controlled by CMV promoter that can express Cre enzyme in mammalian cells, purchased from Addgene, cat.#11916) /Lipo3000 mixed solution (prepared as in Preparation Example 2) was added to each well, incubated in an incubator for 24 hours at 37°C, 5% CO₂. Then, 8 × 10⁵ pfu of MiTDvv-mCherry and MiTDvv-hIL21-mCherry virus were added to well accordingly, and control wells were set up with virus alone and plasmid alone. The place was cross-mixed and continued to incubate for additional 24 hours. After observing the cells completely cytopathic under the microscope, the virus was collected by repeating frozen/thawed procedure for 3 times and centrifugation at 300 g for 5 minutes. The virus was labelled as P0 generation.
2) CV1 cells were placed in five 100 mm petri dishes, such that the cell density reached 80%-90% confluence at next day. The P0 generation virus was 10-fold gradient diluted to 10⁻¹-10⁻⁵ with MEM medium. The MEM medium in the petri dishes was discarded, 3 ml of serious diluted virus solution was added to petri dish accordingly, and the cells were incubated in an incubator for 2 hours at 37°C, 5% CO₂. Upon the incubation, the cells were cross-mixed every 30 minutes, and continued to incubate for 2 hours. The medium was then discarded, and 10 mL of 2% (w/v) agarose gel was added. After agarose firmed, the petri dish was placed back into an incubator for continue culture. After about 48 hours, a single white plaque without red fluorescence was picked into a tube containing 200 µL of sterile PBS, frozen and thawed repeatedly for 3 times to release the virus. 100 µL of virus solution was taken out from each plaque and put in a 24-well plate with CV1 cells (cell density reached 80%) for expansion. After about 48 hours, the cells were completely cytopathic and the viruses were collected and labelled as P1-X (X is the number 1, 2, 3, 4..., representing different virus clones). 200 µL of virus solution was applied from each plaque to extract the genome for PCR assay (sequences of primers zP19 and zP20 are shown in Table 1).
3) Step 2) was repeated once to obtain the P2 generation monoclonal viruses MiTDvv and MiTDvv-hIL21, which were identified by PCR (see Table 1 for sequences of primers zP19 and zP20). After confirming that the sequence is correct by sequencing with primers zP19 and zP20 (sequences of primers are shown in Table 1) (Beijing Tsingke Biotechnology Co., Ltd.), it was stored at -80°C.

PCR and sequencing results showed that the 4 repeated miR199T sequences in the MiTDvv backbone virus has been integrated into the 3' UTR region of the E10R of Dvv-VSC20 vaccinia virus, the mCherry/Zeocin sequence is not contained and the gpt gene is integrated into the TK region so as to inactivate the TK function. The 4 repeated miR199T sequences in MiTDvv-hIL21 oncolytic virus has been integrated into the 3' UTR region of the E10R of DDvv-hIL21 vaccinia virus, and the mCherry/Zeocin sequence is not contained. That is, the recombination of the two kinds of virus got success (Figure 9). The structures of viruses are shown in Figure 10.

### Preparation Example 6: Production and Purification of Oncolytic Viruses

1) HeLa cells were placed in fifty of 150 mm culture dishes, such that the cell density reached about 80% confluence at next day, and they were infected with the No.1 of P2 generation monoclonal MiTDvv backbone virus and the No.1 of P2 generation monoclonal MiTDvv-hIL21 oncolytic virus with 0.2 MOI, respectively. After about 2-3 days, the cells were observed cytopathic feature with string bead shape under a microscope, then cells and culture mediun were harvested with a cell scraper.
2) The cells were centrifuged with 25,924×g for 30 minutes at 4°C, and the supernatant was discarded. The pellet was reconstituted in 20 mL of MEM containing 10% FBS. The suspension was frozen/thawed for three times with liquid nitrogen/autoclaved water, and then centrifuged at 25,924×g for 30 minutes at 4°C. The pellet was washed with 20 mL of PBS, centrifuged at 25924×g for 30 minutes at 4°C. The pellet was resuspended in 10 mL of 10 mM Tris pH9.0, sonicated at 60W and 4°C for 30 seconds, with an interval of 30 seconds, for a total of 3 minutes, centrifuged at 300× g for 5 minutes, and the supernatant was collected for next step. A 13.2 mL of ultracentrifuge tube was taken and added with 6 mL of 36% (w/v) sucrose. 5 mL of virus supernatant was added slowly from the top down, centrifuged at 32,900 × g for 80 minutes at 4°C. The pellet was resuspended in 5 mL of 1mM Tris pH9.0, sonicated for 30 seconds, with an interval of 30 seconds for a total of 3 minutes.
3) Preparation and loading of sucrose gradient purification tube: A sucrose gradient purification tube was prepared by slowly adding 2.2 mL of 40% (w/v), 36% (w/v), 32% (w/v), 28% (w/v) and 24% (w/v) sucrose solutions, respectively, to a 13.2 mL ultracentrifuge tube. Then, 2 mL of the virus suspension obtained in step 2) was slowly added, against the wall of the ultracentrifuge tube, on the liquid surface, centrifuged at 26,000 × g and 4 °C for 50 minutes. After that, a milky white virus band in the middle of the ultracentrifuge tube was visible by naked eyes. A 18G-5mL syringe needle was used to pierce the ultracentrifuge tube so as to absorb the virus band, and the virus was placed in a new ultracentrifuge tube (the volume of the absorbed virus band was recorded as V1).
4) A 2-fold V1 volume of 1mM Tris pH9.0 buffer was added to wash the virus to remove residual sucrose, centrifuged at 32,900g at 4°C for 1 hour. The supernatant was discarded, and the pellet was resuspended in 5 mL of 1mM Tris pH9.0, sonicated at 4°C for 30 seconds, with an interval of 30 seconds for a total of 3 minutes, dispensed in sterile EP tubes at 50 µL/tube and stored at -80°C. At the same time, the virus titer was detected by conventional plaque method. The titer detection result of MiTDvv-hlL21 was 6.4×10⁹ pfu/mL, and the titer detection result of MiTDvv was 1.92×10⁹ pfu/mL.

### Example 1: Selective replication of MiTDvv backbone virus in tumor cells

Normal human umbilical vein endothelial cells HUVEC and human cervical cancer cells Hela were placed into 6-well plates at 3×10⁵ cells/well, and incubated overnight at 37°C, 5% CO₂, such that the cell density reached 70% confluence at next day. 0.1 MOI of MiTDvv virus prepared by the above method was added respectively. After infection for 2 hours, the mediums were replaced with their respective medium containing 5% FBS. After culturing for 24 hours and 48 hours, the cell culture medium and cells were collected, frozen and thawed repeatedly for 3 times to lyse the cells, and the lysate was assayed for viral amount using conventional plaque assays.

The results are shown in Figure 11. Under the infection condition of 0.1 MOI, the replication of MiTDvv virus in HUVEC normal cells was lower than that in Hela tumor cells. Figure 11A shows the viral amount in HUVEC cells and Hela cells 24 hours after infection with MiTDvv backbone virus, respectively; Figure 11B shows the viral amount of 48 hours after the infection of HUVEC cells and Hela cells by MiTDvv backbone virus, respectively; and Figure 11C shows the ratio of replication ability of MiTDvv backbone virus in Hela cells to that in HUVEC cells, wherein at 24 hours and 48 hours after the virus infection, the replication of virus in Hela cells were 50.46 folds and 60.56 folds increase relative to those in HUVEC cells, respectively.

### Example 2: Comparison of in vitro killing effects of MiTDvv backbone virus and DDvv-RFP backbone virus on normal cell lines

Human normal embryonic lung fibroblasts MRC-5 were placed in 96-well plates at 1×10⁴ cells/well and incubated overnight at 37°C, 5% CO₂, such that the cell density reached 80% confluence at next day. DDvv-RFP and MiTDvv viruses prepared by the above method were added at 0.03 MOI and 0.1 MOI, respectively. After infection for 2 hours, the medium was changed with DMEM medium containing 5% FBS. Cell growth chart was detected using the xCELLigence Real-time Label-free Cell Analyzer (RTCA) SP (ACEA, xCELLigenc RTCA SP) for 24 hours (n=6, 2-3 repeated experiments). In the experiment, a group of wells with only cells without virus (no virus infection) was used as a negative control group. The control group underwent the same performance at the same time, and each group had 6 replicate wells. The cell killing rate was calculated the ratio (%) of the viral-treated group to the negative control group.

The results are shown in Figure 12. Under different MOI values, the killing effects of MiTDvv virus on MRC-5 normal cells were significantly lower than those of DDvv virus on MRC-5 normal cells (p<0.001).

### Example 3: Replication comparison of MiTDvv backbone virus and DDvv-RFP backbone virus in normal cells

Human normal embryonic lung fibroblasts MRC-5 were placed into 24-well culture plates at 1×10⁵ cells/well, and incubated in DMEM medium with 2% FBS overnight at 37°C, 5% CO₂, such that the cell density reached 80% confluence at next day. DDvv-RFP virus and MiTDvv virus prepared by the above method were added at 0.1 MOI and 0.3 MOI, respectively. After infection for 2 hours, the medium was replaced with DMEM medium containing 5% FBS. In the experiment, a group of wells with only cells without virus was used as a negative control group. The control group underwent corresponding medium change operations at the same time. After culturing for 24 hours at 37°C and 5% CO₂, the culture medium and cells were collected. The viral genome was extracted using TIANamp viral DNA/RNA kit (TIANGEN, DP315), and the viral A46R gene was detected by QPCR method to detect the viral load. The primer sequences are shown in Table 2. The experiment was repeated 3 times and the average value was taken for statistical analysis.

**Table 2**

| Primer | Sequence 5'-3' | Company |
|---|---|---|
| A46R-F | CAGGGAAACGGATGTATA (SEQ ID NO. 25) | Beijing Tsingke Biotechnology Co., Ltd. |
| A46R-R | TGTGTTACAGAATCATATAAGG (SEQ ID NO. 26) | |

The results are shown in Figure 13. Under different MOI values, the replication of MiTDvv viruses in MRC-5 normal cells were significantly lower than that of DDvv viruses in MRC-5 normal cells (*p<0.01; **p<0.05).

### Example 4: Comparison of killing effects of MiTDvv-hlL21 and DDvv-hlL21 oncolytic vaccinia viruses on normal cells

Human normal embryonic lung fibroblasts MRC-5 were placed into 96-well culture dishes at 1×10⁴ cells/well, such that the cell density reached 80% confluence at next day. MiTDvv-hIL21 oncolytic vaccinia virus and DDvv-hIL21 oncolytic vaccinia virus prepared by the above method were added at 0.3 MOI and 1 MOI, respectively. After infection for 2 hours, the medium was replaced with DMEM medium containing 5% FBS to continue the culture for 12 hours at 37°C and 5% CO₂. Cell growth chart was detected using the xCELLigence Real-time Label-free Cell Analyzer (RTCA) SP (ACEA, xCELLigenc RTCA SP). In the experiment, a group of MRC-5 cells without adding virus was used as a blank control group. The control group underwent corresponding medium change operations at the same time and each group had 6 wells. The experiment was repeated three times or more and the average value was taken for statistical analysis.

The results are shown in Figure 14. The killing effects of MiTDvv-hIL21 and DDvv-hlL21 oncolytic vaccinia viruses on normal human lung fibroblasts MRC-5 showed a dose-dependent increasing trend. The killing effect of MiTDvv-hIL21 oncolytic vaccinia viruses on normal human lung fibroblasts MRC-5 was significantly weaker than that of DDvv-hIL21 oncolytic vaccinia virus on MRC-5 at the same dose (0.3 MOI, p<0.05; 1 MOI, p<0.01).

### Example 5: Replication comparison of MiTDvv-hIL21 and DDvv-hIL21 oncolytic vaccinia viruses in normal cells

Normal human lung fibroblasts MRC-5 were placed into 24-well culture plates at 1×10⁵ cells/well, such that the cell density reached 80% the next day. MiTDvv-hIL21 oncolytic vaccinia virus and DDvv-hIL21 oncolytic vaccinia virus prepared by the above method were added at 0.1 MOI and 0.3 MOI, respectively. After infection for 2 hours, the medium was changed to DMEM medium containing 5% FBS to continue the culture for 24 hours at 37°C and 5% CO₂. Then the culture medium and cells were collected and TIANamp virus DNA/RNA kits were used to extract the viral genome. The viral load was detected by the detection of viral gene of A46R by real-time fluorescent quantitative PCR, and the primer sequences are shown in Table 2. In the experiment, a group of MRC-5 cells without adding virus was used as a blank control group. The control group underwent corresponding medium change operations at the same time. Each group had 3 replicate wells, and the average value was used for statistical analysis.

The results are shown in Figure 15. Under the infection conditions of 0.1 MOI and 0.3 MOI, the replications of MiTDvv-hIL21 oncolytic vaccinia virus in normal cells MRC-5 were significantly lower than that of DDvv-hIL21 oncolytic vaccinia virus in MRC-5.

### Example 6: Replication comparison of MiTDvv-hIL21 oncolytic vaccinia virus in normal cells and in tumor cells

Normal human lung fibroblasts MRC-5 and human non-small cell lung cancer cells A549 were spread into 24-well culture dishes at 1 × 10⁵ cells/well, such that the cell density reached 80% the next day. MiTDvv-hIL21 oncolytic vaccinia virus prepared by the above method was added at 0.03 MOI, 0.3 MOI and 3 MOI, respectively. After infection for 2 hours, the medium was changed to DMEM medium containing 5% FBS to continue the culture for 24 hours at 37°C and 5% CO₂. Then the cells and culture medium were collected, and TIANamp virus DNA/RNA kits were used to extract the viral genome. The viral load was detected by the detection of viral gene of A46R by real-time fluorescent quantitative PCR, and the primer sequences are shown in Table 2. In the experiment, a group of cells without adding virus was used as a blank control group. The control group underwent corresponding medium change operations at the corresponding time, and each group had 3 replicate wells.

The results are shown in Figure 16A, the replication of MiTDvv-hIL21 oncolytic vaccinia virus in tumor cells A549 was significantly higher than that in normal cells MRC-5. The replication ratios of MiTDvv-hIL21 oncolytic vaccinia virus at 0.03 MOI, 0.3 MOI, and 3 MOI in tumor cell A549 to those in normal cell MRC-5 (A549/MRC-5) were 51, 23, and 16, respectively (Figure 16B), wherein the X-axis represents the replication ratio of oncolytic virus in A549 cells to MRC-5 cells (that is, A549/MRC-5), and the Y-axis represents the MOI of virus infection.

### Example 7: In vitro killing effect of MiTDvv-hIL21 oncolytic vaccinia virus on different tumor cell lines

Various types of human tumor cells were spread in 96-well plates at 4000-5000 cells per well. The tumor cells included SK-HEP-1 cells (human liver cancer cells), A549 cells (human non-small cell lung cancer cells), FaDu cells (human head and neck cancer cells), PANC-1 (human pancreatic cancer cells), U251 cells (human glioma cells), LOVO cells (human colorectal cancer cells), MNNG/HOS C1 cells (human osteosarcoma cells). The cells were incubated overnight at 37°C, 5 % CO₂ to reach 70%. Half volume of the medium in each well was aspirated and discarded, and then an equal volume of serum-free medium containing different concentration (0.001MOI, 0.003MOI, 0.01MOI, 0.03MOI, 0.1MOI, 0.3MOI, 1MOI, 3MOI, 10MOI) of MiTDvv-hIL21 oncolytic vaccinia virus prepared by the above method were added to each well. 4 hours after the infection, the culture medium was replaced with 5% FBS-containing cell culture medium, and incubated for 48 hours (MNNG/HOS C1, A549, LOVO, FaDu) or 72 hours (SK-HEP-1, U251, PANC-1) at 37°C, 5% CO₂. The apoptosis of tumor cells was detected by MTT assay (n=6, 2-3 repeated experiments). The control groups of this test were: negative control group (no virus infection) and positive control group (10 µM paclitaxel, purchased from Beijing SL Pharmaceutical Co.,Ltd.), each experimental group and control group were set up with 6 duplicate wells. The cell killing rate (inhibition rate) was the ratio% of the experimental group or positive control group to the negative control group.

The results showed that the killing effect of MiTDvv-hIL21 oncolytic vaccinia virus on tumor cells was dose-dependent (Figure 17). The median inhibition rate (IC₅₀) of MiTDvv-hIL21 oncolytic vaccinia virus on SK-HEP-1 cells, U251 cells, PANC-1 cells, MNNG/HOS C1 cells, A549 cells, LOVO cells and FaDu cells were 0.18, 0.79, 0.54, 0.53, 0.66, 0.68, and 0.29, respectively (Table 3).

**Table 3**

| Cell line | IC₅₀ | |
|---|---|---|
| | 48 hours | 72 hours |
| FaDu | 0.29 | - |
| A549 | 0.66 | - |
| LOVO | 0.68 | - |
| MNNG/HOS C1 | 0.53 | - |
| SK-HEP-1 | - | 0.18 |
| PANC-1 | - | 0.54 |
| U251 | - | 0.79 |

### Example 8: Evaluation of the correlation between the expression level of miR-199 mature form and the cell killing effect of MiTDvv-hIL21 oncolytic vaccinia virus in different cells.

1) Evaluation of the expression level of miR-199 in different tumor cells (note: the expression level of miR-199 in all tumor cells is relative to that in HEK-293T cells): HEK-293T cells, MNNG/HOS C1 cells, SaoS2 cells (human osteosarcoma cells), A549 cells, FaDu cells, SK-BR-3 cells (human breast cancer cells), HepG2 cells (human liver cancer cells), Hela cells (cervical cancer cells), SKOV3 cells (human ovarian cancer cells), MCF-7 cells (human breast cancer cells), C33A cells (human cervical cancer cells), LOVO cells, U251 cells, HCT116 cells (human colorectal cancer cells), SK-HEP-1 cells, CFPAC-1 cells (human pancreatic cancer cells), PANC-1 cells (human pancreatic cancer cells), NCI-H1299 cells (human non-small cell lung cancer cells), and U87MG cells (human glioma cells) were spread on 6-well plates respectively. After the cells were completely full of the well, the culture medium was discarded. 1 mL of Trizol reagent (Invitrogen) was added to extract RNA. 1 µg of RNA was used to synthesize cDNA by using a reverse transcription kit (Tiangen cat# KR116-02), and a fluorescence quantitative PCR reagent kit was used to detect the expression level of miR-199 mature form (hsa-miR-199a-3p or hsa-miR-199b-3p) in tumor cells. A reverse transcription primer miR199-RT was used, which was obtained from the universal stem-loop sequence GTCGTATCCAGTGCAGGGTCCGAGGTATTCGCACTGGATACGAC (SEQ ID NO. 36) combined with inverted repeat sequence of 7 bases at the 3' end of the miR199-3p mature form, as shown in Table 4; the sequences of QRT-PCR primers (synthesized by Beijing Tsingke Biotechnology Co., Ltd.) were shown in Table 4. At the same time, the relative expression amount of miR-199 was calculated using the endogenous gene U6 as an internal reference. The results showed that MNNG/HOS C1 cells and SaoS2 cells were cell lines with high-expressing miR-199 (++,≥10), and FaDu cells, HepG2 cells and SK-BR-3 cells were cell lines with medium-expressing miR-199 (+, ≥1 and <10), other tumor cell lines were all cell lines with low-expressing miR-199 (±, <1) (Figure 18).

**Table 4**

| Primer | Sequence 5'-3' |
|---|---|
| U6-RT | GTCGTATCCAGTGCAGGGTCCGAGGTATTCGCACTGGATACGACAAAATATG (SEQ ID NO.27) |
| U6-F | CTCGCTTCGGCAGCACATA (SEQ ID NO.28) |
| U6-R | CGCAGGGTCCGAGGTATTC (SEQ ID NO.29) |
| miR199-RT | GTCGTATCCAGTGCAGGGTCCGAGGTATTCGCACTGGATACGACTAACCAA (SEQ ID NO.30) |
| miR199-F | GCCGACAGTAGTCTGCACATTGG (SEQ ID NO.31) |
| miR199-R | CGCAGGGTCCGAGGTATTC (SEQ ID NO.32) |

| | |
|---|---|
| Note: U6-RT and miR199-RT are reverse transcription primers; U6-F and U6-R are QPCR primers for U6; miR199-F and miR199-R are QPCR primers for miR199. | |

2) According to the intracellular expression level of miR-199 shown in Figure 18, low-expressing, medium-expressing and high-expressing tumor cell lines PANC-1, SK-HEP-1, FaDu and MNNG/HOS C1 cells were selected respectively for comparing the killing effects of the MiTDvv-hIL21 oncolytic vaccinia virus prepared by the above method: MNNG/HOS C1, FaDu, SK-HEP-1 and PANC-1 cells were spread in 96-well plates and the number of cells in each well was adjusted according to the cell growth rate. After overnight culture, the cell density reached 80%. Then the culture medium was discarded and 100 µL of serum-free culture medium containing 0.1 MOI MiTDvv-hlL21 oncolytic vaccinia virus was added respectively. 4 hours after infection, the culture medium was replaced with 5% FBS-containing cell culture medium to continue incubating for 48 hours. The apoptosis of tumor cells was detected by MTT assay (n=6, 2-3 repeated experiments). The control groups of this test were: negative control group (no virus infection), each experimental group and control group were set up with 6 duplicate wells, the cell survival rate was the ratio% of the experimental group to the negative control group. The results showed that the killing effect of MiTDvv-hIL21 oncolytic vaccinia virus on tumor cells with low-expressing miR-199 was significantly higher than that on tumor cells with high-expressing miR-199 (Figure 19).

### Example 9: Evaluation of the killing effect of MiTDvv-hIL21 oncolytic vaccinia virus on high-expressing miR-199 tumor cells by stable-transfection.

The purpose of this experiment is to further demonstrate the mechanism of the oncolytic virus of the present invention. The TK/VGF double-deleted oncolytic vaccinia virus *per se* has different killing effect on different types of tumor cells. In order to further verify that the MiTDvv-hIL21 oncolytic vaccinia virus of the present invention can be regulated by microRNAs, stably-transfected tumor cells with high-expressing miR-199 (including has-miR-199a-3p and has-miR-199b-3p) were constructed for the comparison between the same type of tumor cells.
1) Construction of lentivirus carrying miR-199: HEK-293 cells (Agilent Technologies) were placed in a 6-well plate with 10% FBS+1% P/S DMEM medium at 4 × 10⁵ cells/well, such that the cell density reached about 70% confluence at next day. After replacing with the 5% FBS+1% P/S DMEM medium, 300 µL of plasmid/Lipo3000 mixed solution (the preparation is the same as described in Preparation Example 2) was added to each well. The plasmids for preparing LV-miR199 lentivirus include: p59-rev, pP60-VSV-G, p61-gag-P01 and LV3-has-miR199-GFP, which were purchased from Shanghai GenePharma Co., Ltd. The LV3-has-miR199-GFP plasmid contains DNA sequence capable of expressing miR199 mature form hsa-miR-199a-3p or hsa-miR-199b-3p : 5'-GATCCGACAGTAGTCTGCACATTGGTTATTCAAGAGATAACCAATGCAGACTACT GTCTTTTTTGAATT-3' (SEQ ID NO.37), and DNA sequence capable of expressing GFP green fluorescence protein that can be used for screening and detection. The plasmids for preparing LV-miRNC lentivirus include: p59-rev, pP60-VSV-G, p61-gag-P01 and LV3-shNC, which were purchased from Shanghai GenePharma Co., Ltd. LV3-shNC is a control plasmid which contains an unrelated control DNA sequence: 5 '-GATCCGTTCTCCGAACGTGTCACGTTTCAAGAGAACGTGACACGTTCGGAGAAC TTTTTTGAATT-3' (SEQ ID NO.38); wherein "LV3" is a serial number of the lentiviral plasmid vector from Shanghai GenePharma Co., and "hsa" indicates it derived from human. Cells were incubated in an incubator for 48 hours at 37°C, 5% CO₂, and then the supernatant was collected, centrifuged at 5000 rpm/min at 4°C for 30 minutes. The supernatant was collected, filtered with a 0.45 µm filter membrane and dispensed into 1 mL per tube, and stored at -80°C. They are Lentivirus LV-miR199 carrying miR-199 and backbone lentivirus LV-miRNC, respectively.
2) Construction of tumor cells with high-expressing miR-199: infecting HCT116 cells with LV-miR199 lentivirus and backbone lentivirus LV-miRNC respectively (the routine operation without description in detail), monoclonal positive cells were selected to construct HCT116-miR199 cell line with stable expression of miR-199 and negative control cell line HCT116-miRNC. The green fluorescence channel of the cytometer was used to detect the expression of GFP (Figure 20A) and PCR method was used for identification (Figure 20B) (it may be adjusted according to the experimental conditions, and therefore not described in detail). The QPCR primer sequences are shown in Table 4. The results of flow cytometry showed that the positive transfection rates of HCT116-miR199 cell line and HCT116-miRNC cell line were 99.91% and 99.64%, respectively. PCR results confirmed that HCT116-miR199 cell line highly expressed miR-199 (including has-miR-199a-3p and has-miR-199b-3p).
3) HCT116-miR199 cell line and HCT116-miRNC cell line were placed in a 96-well plate with 5000 cells per well in 10% FBS medium, and incubated overnight such that the cell density reached 80% confluence at next day. After discarding the medium, 100 µL of serum-free culture medium containing 0.03MOI, 0.3MOI, and 3MOI of MiTDvv-hIL21 oncolytic vaccinia virus prepared by the above method were added, respectively. 4 hours after infection, the culture medium was replaced with 5% FBS-containing cell culture medium, and the cells were incubated for 48 hours at 37°C, 5% CO₂. The apoptosis of tumor cells was detected by MTT assay (n=6, 2-3 repeated experiments). The control groups of this test were: negative control group (no virus infection), each experimental group and control group were set up with 6 duplicate wells, the cell killing rate was the ratio% of the experimental group to the negative control group. The results showed that the killing effect of MiTDvv-hIL21 oncolytic vaccinia virus was significantly reduced in tumor cells with high-expressing miR-199 (Figure 21).

### Example 10: Tumor suppression effect of MiTDvv-hIL21 oncolytic vaccinia virus on colorectal cancer

Human colorectal cancer HCT116 cells in logarithmic growth phase were used to establish the tumor model of NCG mouse, a severe immunodeficient mouse. In the experiment, NCG mice with severe immunodeficiency (obtained from Jiangsu GemPharmatech Co., Ltd) (8 weeks old, female) were used, and each mouse was inoculated subcutaneously with 1 million HCT116 cells in the back of the hind leg. When the tumor volume reached around 100 mm³, the MiTDvv-hIL21 oncolytic vaccinia virus prepared by the above method was injected intratumorally at 5×10⁵ pfu/mouse with an administration volume of 50 µL. There were 4 mice in each group, and the tumor size and body weight of the mice were measured every 3 days. In the experiment, a control group administered with PBS was set. The results showed that MiTDvv-hIL21 oncolytic vaccinia virus effectively inhibited tumor growth (Figure 22A). The relative tumor growth rate (T/C, that is, the ratio value of the tumor volume of the virus-administrated group to the tumor volume of the control group, the value < 40% indicates being effective) of the administration group was less than 40% on and after Day 10 after the administration (Figure 22B). There was no significant decrease in the body weight of the mice throughout the experiment (Figure 22C).

### Example 11: Tumor suppression effect of MiTDvv-hIL21 oncolytic vaccinia virus on human osteosarcoma

Human osteosarcoma MNNG/HOS C1 cells in logarithmic growth phase were used to construct the tumor model of NCG mouse with severe immunodeficiency. In the experiment, NCG mice with severe immunodeficiency (obtained from Jiangsu GemPharmatech Co., Ltd) (8 weeks old, female) were used, and each mouse was inoculated subcutaneously with 0.5 million MNNG/HOS C1 cells in the back of the hind legs. When the tumor volume was about 100 mm³, the MiTDvv-hIL21 oncolytic vaccinia virus prepared by the above method was injected intratumorally at 1×10⁶ pfu/mouse, with an administration volume of 50 µL. There were 4 mice in each group, and the tumor size and body weight of the mice were measured every 3 days. In the experiment, a control group administered with PBS was set. The results showed that MiTDvv-hIL21 oncolytic vaccinia virus also effectively inhibited the growth of MNNG/HOS C1 tumors with high-expressing miR-199 in mice (Figure 23A). The relative tumor growth rate (T/C) was less than 40% on and after Day 8 after the administration (Figure 23B). There was no significant decrease in the body weight of the mice throughout the experiment (Figure 23C).

### Example 12: Tumor suppression effect of MiTDvv-hIL21 oncolytic vaccinia virus on human liver cancer

Human hepatoma SK-HEP-1 cells in logarithmic growth phase were used to construct the tumor model of NCG mouse with severe immunodeficiency. In the experiment, NCG mice with severe immunodeficiency (obtained from Jiangsu GemPharmatech Co., Ltd) (7 weeks old, female) were used, and each mouse was inoculated subcutaneously with 10 million SK-HEP-1 cells in the back of the hind legs. When the tumor volume was about 100 mm³, the MiTDvv-hIL21 oncolytic vaccinia virus prepared by the above method was injected intratumorally at 2×10⁵ pfu/mouse, with an administration volume of 50 µL. There were 3-4 mice in each group, and the tumor size and body weight of the mice were measured every 3 days. In the experiment, a control group administered with PBS was set. The results showed that MiTDvv-hIL21 oncolytic vaccinia virus effectively inhibited tumor growth (Figure 24A). There was no significant decrease in the body weight of the mice throughout the experiment (Figure 24B).

### Example 13: Evaluation of the killing effects of MiTDvv-hIL21 and DDvv-hIL21 oncolytic vaccinia viruses on tumor cells with different expression levels of miR-199

Various human tumor cells were placed in 96-well plates at 4000-5000 cells per well. The tumor cells included MNNG/HOS C1, SaoS2, FaDu, SK-BR-3, HepG2, Hela, SKOV3, C33A, LOVO, U251, HCT116, SK-HEP-1, CFPAC-1 and PANC-1 cells. The cells were incubated at 37°C, 5% CO₂ overnight, such that the cell density reached 70%. The medium was changed to serum-free medium with MiTDvv-hIL21 oncolytic vaccinia virus or DDvv-hIL21 oncolytic vaccinia virus prepared by the above method at 0.3-10 MOI (due to the different sensitivities of various tumor cells to the virus, the MOI explored in the previous *in vitro* experiments was used: 0.3 MOI for MNNG/HOS C1 and FaDu; 1 MOI for LOVO, U251 and SK-HEP-1; 3 MOI for SaoS2, SK-BR-3, HepG2, Hela, SKOV3, C33A, CFPAC-1 and PANC-1; and 10 MOI for HCT116). 4 hours after the infection, the culture medium was replaced with 5% FBS-containing cell culture medium, and the cells were incubated for 48 hours at 37°C, 5% CO₂. The apoptosis of tumor cells was detected by MTT assay (n=6, 2-3 repeated experiments). The negative control group of this test was without virus infection, and each experimental group and control group was set up with 6 duplicate wells. The cell killing rate (cell growth inhibition rate) was the ratio% of the experimental group to the negative control group. The results showed that in tumor cells MNNG/HOS C1 and SaoS2 with high-expressing miR-199, there were no difference in cell killing effects between MiTDvv-hIL21 oncolytic vaccinia virus and DDvv-hIL21 oncolytic vaccinia virus, while in the tumor cells FaDu, SK-BR-3, HepG2, Hela, SKOV3, C33A, LOVO, U251, HCT116, SK-HEP-1, CFPAC-1, PANC-1 etc. with medium-expressing or low-expressing miR-199, MiTDvv-hIL21 oncolytic vaccinia virus exhibited stronger killing effect (Figure 25).

### Example 14: Evaluation of the tumor suppression effects of MiTDvv-hIL21 and DDvv-hIL21 oncolytic vaccinia viruses on tumor-bearing mice with different expression levels of miR-199

Human colorectal cancer HCT116 cells in logarithmic growth phase and human osteosarcoma MNNG/HOS C1 cells in logarithmic growth phase were used to establish the tumor model of NCG mouse with severe immunodeficiency. According to the methods for constructing tumor models described in Examples 10 and 11, human osteosarcoma MNNG/HOS C1 cells with high-expressing miR-199 and human colorectal cancer HCT116 cells with low-expressing miR-199 were inoculated subcutaneously in the back of the hind legs of NCG mice. When the tumor volume was reached about 100 mm³, the MiTDvv-hIL21 oncolytic virus and DDvv-hIL21 oncolytic vaccinia virus prepared by the above method were injected intratumorally, wherein the administration dose for MNNG/HOS C1 tumor-bearing mice was 1×10⁶ pfu/mouse, and the administration dose for HCT116 tumor-bearing mice was 5 ×10⁵ pfu/mouse, with an administration volume of 50 µL. There were 3 mice in each group, and the tumor size and body weight of the mice were measured every 3 days. The results showed that both HCT116 and MNNG/HOS C1 tumor model groups showed relatively good tumor suppression effects. In comparison, in HCT116 tumors with low-expressing miR-199, the tumor suppression effect of MiTDvv-hIL21 oncolytic vaccinia virus was stronger than that of DDvv-hIL21 oncolytic vaccinia virus (Figure 26A). However, in MNNG/HOS C1 tumors with high-expressing miR-199, the tumor suppression effect of MiTDvv-hIL21 oncolytic vaccinia virus was weaker than that of DDvv-hIL21 oncolytic vaccinia virus (Figure 26B).

### Example 15: Evaluation of the tissue distribution of MiTDvv-hIL21 and DDvv-hlL21 oncolytic vaccinia viruses in mice

8-week-old normal C57BL/6N female mice were intravenously administered with 200 µL of PBS containing 1 × 10⁹ pfu/kg of MiTDvv-hIL21 oncolytic vaccinia virus or DDvv-hIL21 oncolytic vaccinia virus prepared by the above method, respectively. There were 5 mice in each group. A group of mice (5 mice) were sacrificed on Day 1 (D1), Day 3 (D3), Day 7 (D7) and Day 15 (D15) after the administration, respectively, and the peripheral blood, heart, liver, spleen, lung, kidney, ovary, and uterus were harvested to detect the distribution of the vaccinia viruses by QPCR method for the viral gene of A46R. The primer sequences are shown in Table 2. The results showed that MiTDvv-hIL21 oncolytic vaccinia virus had lower replication than that of DDvv-hIL21 oncolytic vaccinia virus in ovary, uterus, liver and spleen, and the viral amount of MiTDvv-hIL21 oncolytic vaccinia virus in the lung was similar to that of DDvv-hIL21 oncolytic vaccinia virus. In peripheral blood, the viral amount of MiTDvv-hIL21 oncolytic vaccinia virus was higher than that of DDvv-hIL21 oncolytic vaccinia virus (Figure 27). Neither MiTDvv-hIL21 oncolytic vaccinia virus nor DDvv-hIL21 oncolytic vaccinia virus was detected in the heart and kidney (data are not shown).

## Claims

1. An isolated recombinant oncolytic vaccinia virus, wherein the recombinant oncolytic vaccinia virus is capable of being regulated by a microRNA which has a lower expression level in tumor cells of a mammal than in normal cells of the same mammal, wherein the 3'UTR region of the E10R gene in the recombinant oncolytic vaccinia virus genome is integrated with a target sequence of the microRNA.

2. The recombinant oncolytic vaccinia virus according to claim 1, wherein the microRNA is selected from a group consisting of miR-9, miR-15a, miR-16, miR-26a, miR-27b, miR-29b, miR-30a, miR-32, miR-33, miR-34, miR-95, miR-101, miR-122, miR-124, miR-125a, miR-125b, miR-126, miR-127, miR-128, miR-133b, miR-139, miR-140, miR-142, miR-143, miR-145, miR-181, miR-192, miR-195, miR-198, miR-199a, miR-199b, miR-200, miR-203, miR-204, miR-205, miR-218, miR-219, miR-220, miR-224, miR-345 and miR-375; preferably miR-199a and miR-199b.

3. The recombinant oncolytic vaccinia virus according to claim 1, wherein the recombinant oncolytic vaccinia virus is functionally deficient in the TK gene and/or in the VGF gene.

4. The recombinant oncolytic vaccinia virus according to claim 1, wherein the target sequence of the microRNA is repeated and comprised of 2-8 repeats.

5. The recombinant oncolytic vaccinia virus according to claim 3, wherein the genome of the recombinant oncolytic vaccinia virus is integrated with an exogenous IL-21 gene, and wherein the IL-21 gene is capable of being expressed in tumor cells.

6. The recombinant oncolytic vaccinia virus according to claim 3, wherein the TK gene is rendered functionally deficient via the insertion of an exogenous nucleotide sequence into the TK locus.

7. The recombinant oncolytic vaccinia virus according to claim 5, wherein the exogenous IL-21 gene is inserted into the TK locus, thereby causing functional defect of the TK gene.

8. The recombinant oncolytic vaccinia virus according to claim 3, wherein the VGF gene is rendered functionally deficient by being knocked out or via the insertion of an exogenous nucleotide sequence into the VGF locus.

9. The recombinant oncolytic vaccinia virus according to claim 1, wherein the recombinant oncolytic vaccinia virus is the WR strain or the Wyeth strain.

10. The recombinant oncolytic vaccinia virus according to claim 1, wherein the genome of the recombinant oncolytic vaccinia virus is further integrated with an exogenous screening gene which includes gpt gene and/or LacZ gene.

11. The recombinant oncolytic vaccinia virus according to claim 5, wherein the exogenous IL-21 gene is derived from mouse or human.

12. A pharmaceutical composition, wherein the pharmaceutical composition comprises the recombinant oncolytic vaccinia virus according to any one of claims 1 to 11 as an active ingredient, and a pharmaceutically acceptable excipient.

13. The pharmaceutical composition according to claim 12, wherein the pharmaceutical composition comprises the recombinant oncolytic vaccinia virus at doses of 1 × 10⁵ to 5 × 10⁹ pfu.

14. The pharmaceutical composition according to claim 12, wherein the recombinant oncolytic vaccinia virus is administered by intratumoral injection or intravenous administration.

15. A vector for preparing the recombinant oncolytic vaccinia virus according to any one of claims 1 to 11.

16. The vector according to claim 15, wherein the vector comprises an exogenous IL-21 gene controlled by a promoter.

17. A host cell comprising the vector according to claim 15 or 16.

18. Use of the recombinant oncolytic vaccinia virus according to any one of claims 1 to 11 for the preparation of drugs for the treatment of tumors and/or cancers.

19. The use according to claim 18, wherein the tumors and/or cancers include lung cancer, melanoma, head and neck cancer, liver cancer, intracranial tumor, colorectal cancer, bladder cancer, breast cancer, ovarian cancer, uterus cancer, cervical cancer, gastric cancer, esophageal cancer, kidney cancer, prostate cancer, pancreatic cancer, lymphoid cancer, leukemia, bone cancer, testicular cancer and osteosarcoma.

20. A method for treating tumors and/or cancers, comprising administering the recombinant oncolytic vaccinia virus according to any one of claims 1 to 11 to a tumor and /or cancer patient.

21. The method according to claim 20, wherein the recombinant oncolytic vaccinia virus is administered at dose of 1 × 10⁵ to 5 × 10⁹ pfu, once per day for 1-6 consecutive days, or once every two days for 1-6 consecutive times.

22. The method according to claim 20, wherein the recombinant oncolytic vaccinia virus is administered by intratumoral injection or intravenous administration.

23. The method according to claim 20, wherein the tumors and/or cancers include lung cancer, melanoma, head and neck cancer, liver cancer, intracranial tumor, colorectal cancer, bladder cancer, breast cancer, ovarian cancer, uterus cancer, cervical cancer, gastric cancer, esophageal cancer, kidney cancer, prostate cancer, pancreatic cancer, lymphatic cancer, leukemia, bone cancer, testicular cancer and osteosarcoma.
